Europäisches Patentamt

European Patent Office

Office européen des brevets

(19)

(11) Publication number: **0 433 945 A2**

# EUROPEAN PATENT APPLICATION

(12)

(21) Application number: 90124439.2

(22) Date of filing: 17.12.90

(51) Int. Cl.5: **C12N 15/32**, C12N 15/87

(30) Priority: 18.12.89 US 452526
22.08.90 US 570663

(43) Date of publication of application:
26.06.91 Bulletin 91/26

(84) Designated Contracting States:
AT BE CH DE DK ES FR GB GR IT LI LU NL SE

(71) Applicant: SANDOZ LTD.
Lichtstrasse 35
CH-4002 Basel(CH)

(84) BE CH DK ES FR GB GR IT LI LU NL SE

Applicant: SANDOZ-PATENT-GMBH
Humboldtstrasse 3
W-7850 Lörrach(DE)

(84) DE

Applicant: SANDOZ-ERFINDUNGEN
Verwaltungsgesellschaft m.b.H.
Brunner Strasse 59
A-1235 Wien(AT)

(84) AT

(72) Inventor: Jellis, Cindy Lou
3 Brian Drive
Londoberry, New Hampshire 03051(US)
Inventor: Beerman, Noah Daniel
Seven Harrison Avenue
Cambridge, Massachusetts 02140(US)
Inventor: Piot, Jean-Christophe
271 Stanford Avenue
Menlo Park, California 92025(US)

(54) Insecticides produced by transformation of B.t. Kurstaki with heterologeous genes.

(57) Particularly effective B.t. insecticides are obtained by culturing to the sporulation stage novel transformant obtained on transforming B.t. kurstaki H.D. 562 with DNA expressing in the B.t. certain mutant endotoxin genes. Such transformants and other B.t. transformants generally may be obtained in high transformation frequencies by the electrotransformation of B.t. cells while in a hypertonic state followed by maintaining the cells in a hypertonic medium for a time sufficient to obtain intact cells. Transformation of B.t. tenebrionis and B.t. aizawai is also described.

EP 0 433 945 A2

## IMPROVEMENTS IN OR RELATING TO ORGANIC COMPOUNDS

Delta-endotoxin proteins, produced by the sporulating bacterium Bacillus thuringiensis (B.t.) as protein crystals at the end of its vegetative growth, are a potent insecticide against specific insects. The genes responsible for the production of the endotoxin are found on one or more plasmids within the natural B.t. cell. Certain of these plasmids have been isolated, the endotoxin producing genes located and excised, the genes sequenced and the amino acid sequence (structure) of the endotoxins deduced. The precise amino acid structure of the toxic portion of certain endotoxins produced by the wild type organisms is therefore also known, and it has been determined that the endotoxin is a precursor molecule which, it is generally believed, is cleaved by proteases in the insect gut to release the toxic portion. Truncated molecules, ie. those shorter than the full length endotoxin, have been demonstrated to be active. U.K. Patent Application No. 2216127A (published October 4, 1989) and its foreign counterparts describe full length and truncated endotoxin molecules which are mutated in the active toxin portion and which are indicated to have up to five times increased toxicity.

### BRIEF SUMMARY

The present invention concerns particularly active B.t. kurstaki insecticides provided when certain B.t. kurstaki cells are transformed with DNA carrying an expressible gene encoding certain mutant B.t. endotoxins described in said U.K. Patent Application No. 2216127A herein also "prior application".

More particularly, new B.t. type insecticides having potent and desirably insecticide activity are provided when an expressible exogenous gene encoding an endotoxin having one or more of the mutations described as p26-3 and p98c1 resp. pBT 26-3 and pBT 98c1 in said prior application are transformed into and harbored by the known B.t. kurstaki strain type H.D. 562.

The present invention also concerns the improved process for the electrotransformation of B.t. cells in which the cells are grown and transformed in a hypertonic state and maintained in a hypertonic aqueous media following transformation for a time to sufficient to obtain intact cells. High transformation efficiencies are obtained.

The present application further concerns B.t. Tenebrionis and B.t. aizawai may also be transformed by a process that employs DNA conditioning and the resulting transformants.

### DESCRIPTION OF THE DRAWINGS

The present invention will be more particularly evident from the following description and accompanying drawings in which:

Figure 1: illustrates the shuttle plasmid or vector pUC:BiB produced from plasmids pUC18 and pBC16.1.

Figure 2: illustrates the expression vector pBEV210-TL.

Figure 3: illustrates the expression vector pBt1000.

Figure 4: represents a graph showing percent survival vs. electrotransformation voltage applied for transformation of B.t. crystal minus cells (Cry B).

Figure 5: represents a graph showing percent survival vs. electrotransformation efficiency of Cry B cells.

Figure 6: illustrates the expression vector pBT 2000.

The vector pK8-1 herein was deposited with the National Regional Research Laboratory at Peoria, Illinois, USA and received Repository No. NRRL B-15967 on April 24, 1985 and the vector pBT210 was deposited on February 19, 1988, and received Repository No. NRRL B-18330.

### DETAILED DESCRIPTION

As disclosed in said U.K. Patent Application No. 2216127A and its foreign counterparts, there are a number of mutations or amino acid changes which can be made in a B.t. endotoxin, whereby enhanced activity was indicated. Table A hereto (which is also given and described in said prior applications) gives the full length DNA and amino acid sequences of a B.t. endotoxin of B.t. wuhanensis and, as recognized in the art, the encoded amino acid sequence for the active portion of the protein and beyond, in particular from the N-terminus up to at least the indicated Kpn I site in the protoxin portion, is the same as found in other B.t. species and varieties, in particular B.t. Kurstaki HD-1. Differences beyond the Kpn I site are also

2

relatively few. Hence, the sequence given in Table A fairly represents a common endotoxin sequence, particularly in its active portion, and has, particularly as regards the active portion, a high degree of homology or similarly to other B.t. endotoxin. Table A numbers the amino acid of the full sequence 1 to 1181 in parenthesis below the amino acid. Nucleotides in the structured gene are numbered (not in parenthesis) above the line in which they appear and the last digit in the number stands above the nucleotide to which the number applies. Within the numbered sequences indicated above a portion thereof is separately or sub-numbered m-1 through m-116 for amino acids and n-1 through n-348 for the nucleotides for such amino acids, to more particularly indicate a highly conserved region in which certain of mutations described in said prior applications are located. Certain restriction sites relevant to the nucleotide sequence are shown by a line above the nucleotides involved in the restriction sites with a footnote designation of the particular site. The toxic portion of the endotoxin shown in Table A as recognized in the art involves the amino acid sequence beginning at amino acid position 1(Met) and extending through amino acid position 610 (Thr).

As indicated from the results in Example 7, mutations from two of those previously described and designated "26-3" and "98c1" in said prior applications were indicated to have unexpectedly beneficial influence on insectical activity when transformed into the known endotoxin-producing B.t. Kurstaki strain H.D. 562. These involved mutations, as described in the prior application are as indicated in the following Table B:

## Table B

| Mutant | Position No. Full Length | "m" Position Number | Change In Endotoxin Area | |
|---|---|---|---|---|
| | | | Nucleic Acid | Amino Acid |
| | 119 | m-30 | GCA --> ACA | Ala --> Thr |
| p 26-3 | 130 | m-41 | ATG --> ATA | Met --> Ile |
| | 201 | m-112 | GGC --> GAC | Gly --> Asp |
| p 98c1 | 188 | m-99 | ACT --> TCT | Thr --> Ser |

Hence, by present invention there may also be produced particularly effective insecticids of the B.t. type by growing to the sporulation stage at which endotoxins are expressed or produced cells of a Bacillus thuringiensis kurstaki strain H.D. 562 which comprises or harbors its native plasmids for expression of its native B.t. endotoxins and also harbors or is transformed with heterologous DNA comprising an orgin of replication in said B.t. cells and DNA encoding a B.t operable gene for theexpression of a mutant B.t. endotoxin protein having insecticidal activity against tobacco budworm larva upon ingestion by the insect, the structural gene DNA for said mutant protein characterized by having a DNA portion coding for an amino acid sequence having substantial amino acid homology with the 116 amino acid sequence encoded by the DNA beginning at position m-1 and extending through postion m-116 in Table A hereof, said position numbers applying to such homologous sequence independent of any deletions or additions therein compared to said 116 amino acid sequence, and said DNA portion being further characterized by any one or more of the following amino acids being coded for by said DNA at the indicated amino acid reference positions: a) at position m-30 any natural amino acid except Ala; b) at position m-41 any natural amino acid except Met; c) at position m-99 any natural amino acid except Thr; and d) at position m-112 any natural amino acid except Gly.

The indicated point mutations may be applied to endotoxin protein sequences produced by Bacillus thuringiensis varieties and subtypes, which sequences are insecticidal active against Lepidopteran larvae as indicated by the Tobacco Budworm Assay herein described when containing the indicated 116 amino acid conserved sequence or a sequence which is highly homologous therewith or essentially an equivalent thereof, including preferably protein endotoxin sequences which are of the natural full length type or substantially full length, but also those which are truncated by removal of all or a part of downstream protoxin or inactive portion thereof (which extends upstream from the endotoxin normal C-terminus to the point of cleavage in the insect gut); and even those which may be truncated from the normal C-terminus upstream and back into the active portion of the endotoxin. Endotoxins from B.t. Kurstaki and B.t. Wuhanensis have the identical 116 amino acid conserved region and others have or can be expected to have the same 116 amino acid sequence or a largely homologous equivalent thereof. For example, endotoxins from B.t. Sotto, B.t. Kurstaki HD-73 (strain), and B.t. Galleriae are already also known to produce endotoxins with the idential 116 amino acid sequence even though some of these differ to at least some extent, and in some cases significantly, in both the balance of the toxic portion of the endotoxin and in the protoxin section. Others such as B.t. Kurstaki HD-1 Dipel (a commercial substrain) have one amino acid change in the indicated 116 amino acid sequence (m-59 is Leu coded for TTG) and other changes/deletions/additions in other sequence portions. This and others found to have a single or multiple changes but amino acid homology of at least about 70% to said 116 amino acid sequence may have one or more of the indicated mutant changes made to the amino acids therein which corresond (identically) to the amino acid in said 116 amino acid non-mutated sequence, particularly when the amino acid to be changed has on each of its sides 2 and preferably 4 other amino acids which also correspond (identically) to those in the 116 amino acid sequence. The indicated mutations may be made to corresponding amino acids in homologous series which essentially contain deletions or additions such that the sequence itself is shorter or longer that the indicated 116 amino acid reference sequence. In such cases, each corresponding amino acid in the sequence to be changed will be assigned a position number which is the same as the amino acid to which it is found to correspond (identically) in the indicated 116 amino acd reference sequence, e.g. assigned position number m-5, m-6, etc. In such cases, deletions existing in the sequence to be changed will be counted as actually present and additions in the sequence to be changed will simply not be counted. Hence, amino acid positioning assignment can be said to be made independent of deletions or additions in such a homologous sequence.

Preferably, the homologous amino acid sequences into which the mutant changes may be substituted are those which are coded for by structural DNA to which DNA from either the sense or antisense strand (or double strand) of the 348 nucleotide oligomer having the nucleotide sequence beginning at position n-1 and extending through position n-348 in Table A will hybridize under stringent hybridizing conditions when the homologous sequence to be mutated has its amino acids which correspond to those in the referenced 116 amino acid sequence coded for by the same codon as the corresponding amino acid in the reference sequence. Procedures for preparing such a tagged hybridization probe are well known in the art. Stringent hybridizing conditions are those in which hybridization is effected at $60^\circ$ C in 2.5X saline citrate buffer (a.k.a. SSC buffer) followed merely by rinsing at $37^\circ$ C at reduced buffer concentration which will not affect the hybridizations which take place.

More preferably, the mutations are made in amino acid sequences which have no more than 1, 2 or 3 amino acid differences from those in the 116 amino acid reference sequence, most preferably a sequence which is identical to the reference sequence.

It is already clearly indicated in the art that the 116 amino acid reference sequence may form a portion of otherwise substantially modified or different endotoxin protein sequences which have insecticidal activity against Lepidopteran larvae, and other modifications outside of the reference sequence and perhaps even within the reference sequence will most certainly be uncovered as knowledge of the art unfolds. Hence, the sequences bordering the required mutated sequence portion which is analogous to the 116 amino acid reference portion may vary to a considerable extent and need only be sufficient to provide insecticidally active endotoxin protein (eg. insectically active against the Tobacco Budworm). Thus, the amino acid sequence upstream from the mutated portion may be shortened or lengthened or itself mutated relative to the sequence shown in Table A, but will generally begin with methionine and is most preferably highly homologous (70%) or identical to that shown in Table A. Similarly, the portion downstream from the required mutated sequence portion may vary widely and be shortened or lengthened relative to the balance thereof shown in Table A up to its point of cleavage in the insect gut, and of course may or may not be further extended to form a protoxin or inactive portion subject to cleavage in the insect gut to provide an insecticidally proitein toxin. It is judged usually preferred to employ or produce fuller length sequences which are the same as or mimick the native type at least in terms of the opportunity to achieve an endotoxin protein folding capablity similar to that of its native capability, or an improved full length folding effect. Preferably, the fuller length sequence into which the mutations are made will have at least 70% amino acid homology to the amino acid sequence 1 to 1181 in Table A or the double stranded DNA shown in Table A as encoding said 1 to 1181 amino acid protein will hybridize under stringent conditions to the fuller length mutated sequence. Accordingly, the structural gene for said mutant protein will hybridize under stringent conditions to DNA having the coding sequence for the 1181 amino acid endotoxin as set forth in Table A hereof. More preferably, the mutated DNA will code otherwise for the 1181 amino acid protein in Table A, or for a functional equivalent of the mutant protein which substantially provides the advantages thereof in H.D. 562.

In general, for purposes of this application, the DNA to be mutated will code for an endotoxin having activity against the tobacco budworm as would be recognized in the art or when not so apparent may be determined by assay using, for example, the non-mutant gene transformed into Cry B and assaying for activity compared to the essentially inactive untransformed Cry B in a standard budworm assay as described in Example 7, hereof, while such activity will be indicated when providing an $LD_{25}$ at the highest 10% culture concentration, the preferred substrates for mutation will have at least about the budworm activity provided by the vector pBT1000 (herein described) when transformed into Cry B.

Preferably, the mutant amino acid at m-30 is Thr, at m-41 is Ile, at m-99 is Ser and at m-112 is Asp.

As regards the three mutations in the mutated sequence p26-3, one or two of these may be omitted but preferably all three will be used together.

B.t. kurstaki is well-known and exists in several varieties or strains, the taxonomic distinctions among which are established. Mutants strains which are essentially substrains are also known. The B.t. kurstaki strain H.D. 562 is well-known and publically available from the National Regional Research Laboratory at Peoria, Illinois, U.S.A., under the Accession No. NRRL H.D. 562. Desirably, the mutant-containing DNA is transformed into cells of the well known B.t. kurstaki H.D. 562 substrain which are of the commercial JAVELIN[R] substrain. Spores for growth and transformation of such preferred substrain may be obtained from the commercial product.

The B.t. kurstaki cells transformed with or harboring the mutated endotoxin genes as above identified are stable when maintained in the presence of anti-biotic against which the cells express resistance, eg. tetracycline for which resistance is expressed by the plasmid carrying the mutant endotoxin gene. The cells produce at sporulation a biomass which comprises spores of the B.t. along with the native endotoxins produced by the untransformed cells and the mutant endotoxin protein encoded by the DNA inserted on transformation. Such spore-containng biomass and concentrates thereof can be understood as broadly comprising a mixture of the expressed endotoxins which is new and in which the endotoxins are indicated to associate to provide particularly desirably insectical activity by way of level and spectrum of activity, and in particular substantial enhancement of activity against Spodoptera. A further aspect of the present invention therefore is an insecticidal composition comprising a mixture of endotoxin proteins expressed at the sporulation stage by cells in accord with this invention.

The new B.t. kurstaki transformants may be prepared employing certain plasmids and electrotransformation procedures herein described.

Plasmids

Plasmid vectors, which may be used to transform two taxonomically different bacterial hosts, wherein said first host is E. coli and said second host is the Bacillus species, comprise:
i) a region of DNA enabling replication of the vector in a first bacterial host,
ii) a region of DNA enabling replication of the vector in a second bacterial host,
iii) means for selecting transformed first and second hosts,
iv) a region of DNA enabling gene expression in a first bacterial host,
v) a region of DNA enabling gene expression in a second bacterial host,
vi) a DNA sequence which upon expression encodes a B.t. DET.

In one embodiment of such a plasmid, efficient means are provided for deleting a sequence of DNA from the plasmid, such sequence containing the sequence enabling replication in the first host, prior to the transformation of the Bacillus host if such sequences are undesirable.

A vector containing an origin of replication operable in E. coli, and means for selecting E. coli cells transformed with the vector; an origin of replication operable in organisms of the Bacillus species, and means for selecting Bacillus cells transformed or transfected with the plasmid and a DET DNA sequence, in association with both E. coli and B.t. regulatory sequences, may be used to transform both the E. coli and Bacillus hosts, such that the DET sequence may be successfully expressed in both the hosts.

The delta endotoxin sequence for insertion into the plasmid may be a DNA sequence of any of the B.t. varieties or subtypes, which, upon expression in both E. coli and a cell of the Bacillus species, encodes a delta endotoxin protein. Suitable examples of such are the sequences of the natural full length type or substantially full length, and those which are truncated by removal of all or a part of downstream protoxin or inactive portion thereof (which extends upstream from the endotoxin normal C-terminus to the point of cleavage in the insect gut) and even those which may be truncated from the normal C-terminus upstream and back into the active portion of the endotoxin. Sequences which upon expression encode a fusion protein between, for example, the endotoxin proteins of different B.t. strains or DNA sequences into which selective mutations have been engineered to alter the amino acid sequence of the natural endotoxin sequence are also suitable for expression in the plasmid of the invention.

The full length endotoxin structural gene from Bacillus thuringiensis var wuhanensis (B.t.w.) is incorporated, for example, in plasmid pBT210, described in U.K. Patent Application No. 2216127A supra, and publicly available from the Agricultural Research Culture Collection (NRRL), Peoria, Illinois, under Repository No. B-18330. This plasmid is a fully competent E. coli expression vector and comprises an E. coli promoter, a B.t. ribosome binding site and a gene for chloramphenicol resistance. The 610 amino acids of the active portion of the encoded B.t. endotoxin and extending into the protoxin region (at least up to the KpnI site in encoding DNA in pBT210) is identical to the corresponding sequence of a CryI A(b) type gene cloned from B.t. var kurstaki (B.t.k.) HD-1 (see Hofte & Whitely, Microbiological Reviews, June 1989, Vol. 53, No. 2, pages 242-255 for classification of cells). There is also substantial homology in the balance of the protoxin between pBT210 and B.t.k. HD-1.

A truncated endotoxin sequence is contained, for example, in the plasmid prAK, described in U.K. Patent Application No. 2216127A supra, and publicly available from the Agricultural Research Culture Collection (NRRL), Peoria, Illinois, under Repository No. B-18329. prAK is a fully competent expression vector for E. coli and includes an ampicillin resistance gene, an origin of replication and operator sequences including an E. coli promoter. The E. coli operator sequence, as found in both pBT210 and prAK, is described in U.S. 4,721,671, and contains a promoter sequence, ribosome binding site (RBS) and a DNA coding sequence, the latter hereinafter being referred to as the E. coli gene. It also includes, in proper reading frame coordination with the promoter, a DNA sequence which is found in the wild type B.t.k. strain HD-1 (a Cry 1 A(b)type gene) from a portion of the 5.3kb Hind III class segment as reported, for example, by Kronstad et al., 1983, J. BACTERIOL 154:419-428). The mature sequence has been shortened to code for a truncated B.t. endotoxin which includes the entire native toxic portion extending from amino acid position one to amino acid position 610 and further extending into the protoxin portion to end with amino acid position 723. Downstream, or 3', of this it includes a short DNA sequence of 54bp following the triplet for amino acid 723 and which is itself terminated by a stop signal. This 54bp sequence originates from the plasmid pBR322. Upstream of the coding sequence and downstream of the E. coli gene, prAK contains a sequence which includes a B.t. ribosome binding site. The upstream regulatory sequences and the DNA sequence coding for the promoter sequence and the DNA sequence coding for the first 610 amino acids of the active portion of the endotoxin are identical to the B.t.w. endotoxin sequence contained in plasmid pBT210, above. Upstream of the coding sequence, the sequence of prAK is virtually identical to that of pBT210, with a few insignificant nucleotide changes, whose presence is due to the different ligation procedures used in the construction of the two plasmids.

The full length endotoxin structural gene from Bacillus thuringiensis var. wuhanensis (B.t.w.) is described in U.K. Patent Application No. 2216127A supra, and publicly available from the Agricultural Research Culture Collection (NRRL), Peoria, Illinois, under Repository No. B-18330. This plasmid, (pBT210), is a fully competent E. coli expression vector and comprises an E. coli promoter, a B.t. ribosome binding site and a gene for chloramphenicol resistance.

Mutant analogues of both the WT full length structural B.t. DET and the WT truncated B.t. DET are described in U.K. Patent Application No. 2216127A supra. Any of the mutant sequences described therein, or sequences containing other mutations, may suitably be used in the plasmid of the invention. Hence, full length mutant sequences, such as pBT26-3 and pBT-C are contained in plasmid pBT210, above, and may readily be excised from such plasmids for use in the vector of the invention.

Preferably, the DET DNA sequence is associated with regulatory sequences, which control expression in the two host bacterial cells. The 5' regulatory sequences preferably include a promoter sequence operable in E. coli and cells of the Bacillus species. The sequence illustrated below, which is the naturally occurring regulatory sequence of the DET gene, is a particularly suitable B.t. operator sequence, containing sites for initiation of RNA synthesis during early (B.t.I) and late (B.t.II) stages of sporulation, a ribosome binding site (RBS), from which translation of the mRNA initiates, and an initiation codon (Met). [Wong, et al., J. Biochem. 1983 258(3):1960-1967]. A B.t. sequence, such as that depicted below, is also functional in E. coli, resulting in expression of the DET protein in E. coli cells at a low level.

```
HpaI              A & T rich region
GTTAACACCCTGGGTCAAAAATTGATATTTAGTAAAATTAGTTGCACTTTGTGCATTTTT

      B.t. II         B.t. I
TCATAAGATGAGTCATATGTTTTAAATTGTAGTAATGAAAAACAGTATTATATCATAATG
                  RBS           Met
AATTGGTATCTTAATAAAAGAGATGGAGGTAACTT ATG .....
```

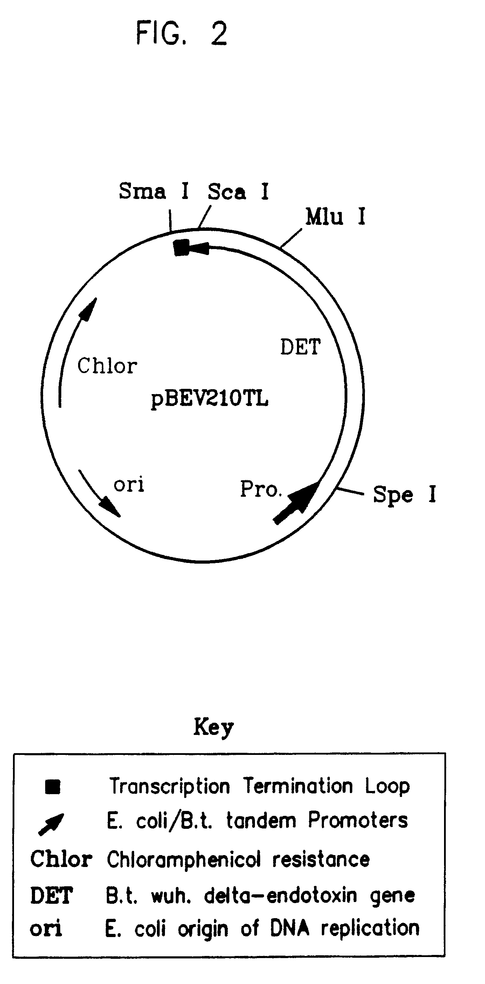

A DNA fragment containing the above sequences, as well as coding sequences for the first 450 amino acids of the Bacillus thuringiensis var. kurstaki strain HD-1 delta endotoxin may be obtained from, for example, the plasmid pK8-1, as described in European Patent Application No. 206 613 A2 published 30 December 1986, which plasmid is publicly available from the Agricultural Research Culture Collection (NRRL), Peoria, Illinois under Repository No. B-15967. Plasmid pK8-1 is a pBR322 based plasmid containing a portion of the B.t.k. DET gene. The DET on expression reacts positively with anti-serum prepared against the 130Kd protoxin and is toxic to Heliothis virescens larvae in an insect toxicity assay. The fragment as described above, may be cloned into a plasmid harbouring a promoter operable in E. coli and directing expression of a DET sequence in E. coli (cf. Figure 2), such as, for example, prAK, described above. Thus, by specific endonuclease digestion, a region of the prAK DNA containing the 5' coding sequences of the DET and the 3 portion of the E. coli portion of the E. coli gene, may be replaced with the DNA sequence of pK8-1, described above. The 3' junction formed between the prAK and pK8-1 fragments is within the B.t. DET coding sequences and the correct reading frame is maintained by the insertion, such that the toxin gene is exactly as it is found in prAK. By virtue of digesting prAK downstream of the E. coli promoter and RBS found therein, i.e. at a site within the E. coli gene, and ligating the pK8-1 fragment to this promoter-containing prAK fragment, the prAK-pK8-1 5' junction results in tandem E. coli/B.t. promoters. Tandem E. coli/B.t. promoters, as used herein, means the E. coli promoter, RBS and the 5' region of the E. coli gene, in tandem with the B.t. promoter. Translation of the E. coli gene results in production of 24 amino acids before this prAK-pK8-1 5' junction is reached. During expression in E. coli, translation will start at the ribosome binding site in the E. coli promoter and will carry on through the prAK-pK81 5' junction into the B.t. promoter sequence for a total of 7 amino acids before a stop codon (TGA) is randomly generated. Although the ribosomes will fall off at this stop codon, they recognize the B.t. ribosome binding site downstream of this and bind the mRNA, such that, in E. coli, the DET is produced as a non-fusion protein with the correct amino terminus shown below, typically at a level of 2-5% of total cellular protein.

```
             NruIHpaI                    STOP
(E. coli gene) T CGA ACA CCC TGG GTC AAA AAT TGA .....


... (115 bases) ... AGATGGAGGTAA CTT ATG .......
             B.t. RBS          (Start B.t. DET)
```

The plasmid produced by a ligation such as that described above will thus contain host specific promoters, for E. coli and Bacillus species, and also a truncated DET sequence of 723 amino acids, as found originally in prAK.

As described above, the plasmid pBT210 contains the full length structural DET gene, with an associated operator sequence, as in prAK, operable in E. coli. To obtain a full length structural DET gene in association with tandem E. coli/B.t. promoters, a fragment of pBT210 containing the E. coli PBS, and 5' portion of the E. coli gene and the sequence coding for the first 450 amino acids of the B.t. DET may be excised and replaced with a fragment from a plasmid, such as that produced by the ligations described above, containing the tandem E. coli/B.t. promoter sequences and a region encoding the identical 450 amino acids of the DET protein. In this manner the correct reading frame for the DET will be retained and the resulting plasmid will express the full length structural DET in E. coli.

Described in more detail in the Examples, a specific example of the above process can be summarized as follows: plasmid prAK is digested with Nrul and Sstl. The Nrul site, at position 575, is within the E. coli gene sequence, downstream of the E. coli promoter and RBS, and the Sstl site is within the DET coding sequence. The larger fragment (3.9kb) is isolated. Plasmid pK8-1 is digested with Hpal and Sstl. The Hpal site is at the 5' end of the B.t. promoter and the Sstl site is within the DET coding sequence, this site being identically positioned with that in prAK. The 1.5kb fragment thus produced is isolated and ligated with the 3.9kb prAK fragment. The blunt end digestion/ligation at the Nrul and Hpal sites causes destruction of these two sites. The resulting hybrid plasmid, pBEV100, containing the E. coli operator portion from prAK and C-terminal DET sequence, B.t. promoter and N-terminal DET sequence of pK8-1, is digested with Hpal and Sstl. The Hpal site is located downstream of the E. coli promoter, between this and the RBS, at position 448. Plasmid pBT210 is likewise digested with Hpal and Sstl, the Hpal site (at position 448) again lying between the E. coli promoter and the RBS, and the larger, 6.9kb, fragment of pBT210 ligated with the smaller, 1.6kb, fragment of pBEV100. The resulting plasmid, pBEV210, contains the E. coli operator and B.t. promoter exactly as in pBEV100, in association with the full length structural DET gene sequence. The ligation at Hpal and Sstl restores these sites.

Further regulatory sequences which are preferably associated with the DET coding sequence include the 3' transcription termination loop (Wobiko, et al., 1986 DNA 5:305-314). This regulatory element, consisting of a sequence of dyad symmetry, is found in naturally occurring B.t. DET genes after the coding portion of the gene and is thought to lead to increased mRNA stability. Complementary oligonucleotides which code for this regulatory element may be cloned downstream, or 3', of the DET coding sequence. For example, with plasmid pBEV210, the production of which is described above, a partial digestion with Pvul creates a linear DNA molecule, the Pvul site, at position 4444, being immediately downstream of the full length structural DET gene sequence contained in pBEV210. Complementary oligonucleotides coding for the 3' transcription termination signal, with Pvul sticky ends may be ligated into this Pvul digested pBEV210, thereby inserting the 3' signal and forming plasmid pBEV210-TL. With the correct orientation of the oligonucleotides, the Smal site found therein, will be situated at position 4490, immediately upstream of the 3'. Pvul ligation site.

The DNA sequences which permit a plasmid to act as an autonomous replicon in its host cell include the origin of replication. Such sequences are host specific, in that a sequence which will allow a plasmid to replicate in a bacterium of one genus will typically not enable replication of that plasmid in a bacterium of a different genus. Many plasmids which are capable of self-replication in E. coli and which may be used in the formation of the plasmid of the invention, are known in the art, for example pBR322, pUC, pk and derivatives thereof. (Plasmids pK18 and pK19 are described, for example, in Pridmore 1987 Gene 56:309-312). In the construction of the plasmid of the invention, it is preferred that sequences derived from the pUC derivatives pUC18 or pUC19 (Bethesda Research Labs) are employed, and more preferably pUC18. pUC18 is a cloning vector with a total size of approximately 2.7kb and contains a gene conferring ampicillin (amp) resistance on a host transformed with the plasmid. It further contains a unique EcoR1 restriction endonuclease recognition site.

An example of a plasmid which replicates in Bacillus sp. is pBC16.1, described by Perkins and Youngman, 1983 J. Biochem. 155(2):607-615. This plasmid originates from a German isolate of B. cereus and has a total size of approximately 2.9kb. The plasmid also carries a gene encoding tetracycline (tet) resistance. There is a unique EcoR1 restriction endonuclease recognition site into which may be cloned DNA sequences of interest.

By a simple digestion and ligation procedure, utilizing a restriction site unique to both vectors, plasmids pUC18 and pBC16.1, for example, may be combined, such that the resulting clone contains origins of replication permitting autonomous replication in both E. coli and Bacillus species, an amp resistance gene and a tet resistance gene for selection of transformed hosts.

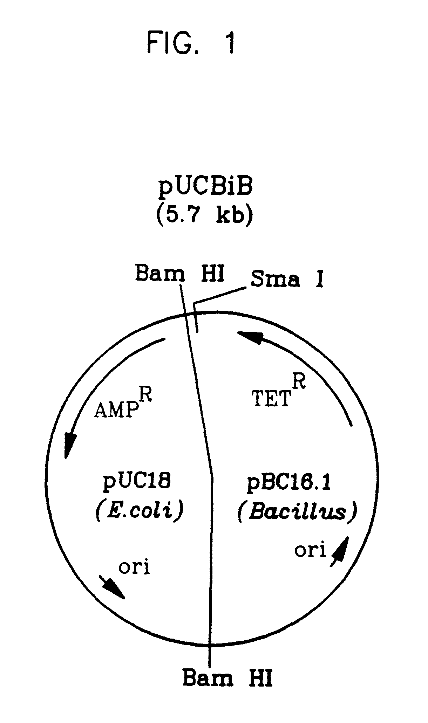

The shuttle vector pUC:B is produced from the ligation of pUC18 and pBC16.1. One of the EcoR1 sites at the junction of these two original plasmids can be changed to, for example, a BamHl site (c.f. Figure 1 and Example 1). This step is done prior to cloning in the DET gene sequences. The resulting plasmid is referred to as pUC:BiB and shown in Figure 1.

For expression of DET genes in E. coli and Bacillus sp., the DET DNA is cloned into pUC:BiB. Preferably the PET DNA sequence will consist of the full length structural gene in association with 5' and 3' regulatory sequences, including the B.t. promoter and 3' transcription termination to make pBEV210TL as shown in Figure 2.

The DET coding and regulatory sequences contained in pBEV210-TL, described above, are preferably cloned into a restriction endonuclease site within the multiple cloning site, upstream of the BamHl site of the shuttle vector pUC:BiB. A suitable site for such insertion is the Smal site. The resulting plasmid, termed pBt1000, is thus formed by excising a Hpal-Smal fragment from pBEV210-TL and inserting this into the Smal site of pUC:BiB. The Hpal digestion occurs at position 448 of pBEV210-TL, and is thus at the site used in the formation of pBEV210-TL. Therefore, the Hpal-Smal fragment inserted into the pUC:BiB vector contains the RBS from the E. coli operator, 5' portion of the E. coli gene, B.t. promoter and the sequence encoding the full length structural DET protein, up to and including the 3' transcription termination signal. pBt1000 thus contains the above sequences, an origin of replication for E. coli and means for selection of transformed E. coli hosts, and an origin of replication for Bacillus cells with means for selection of transformed Bacillus hosts. This plasmid may be used to amplify the total DNA in E. coli and express the DET in E. coli, and to transform Bacillus cells and express the full length structural B.t. DET in Bacillus cells. Because of the presence of a single Bam HI site in the multiple cloning site of pUC18, and the change of the EcoRI site opposite the multiple cloning site to a Bam HI, a single Bam HI endonuclease digestion will enable removal of virtually all of the pUC DNA after amplification of the vector DNA in E. coli if desired. This step is followed by isolation of the larger Bam HI fragment containing pBC16.1 and DET coding and regulatory sequences, religation to form a closed circle and transformation directly into Bacillus.

This DET expression plasmid, pBt1000 may also be used in the construction of similar plasmids containing mutant DET genes. Any of the full length mutant sequences described in said U.K. Patent Application No. 2216127A and published foreign counterparts may be readily prepared and used. For example, the 2.6 kb Mlul/Spel fragment of pBT-98c1 or pBT26-3 may be ligated with the 7kb Mlul/Spel fragment of pBT1000. Plasmids constructed from such mutated sequences are described in more detail in the Example 4, hereinafter.

Any of the above plasmids, or others constructed to contain a DET DNA sequence, (with associated regulatory sequences) E. coli ori and B.t. ori, may be used to transform an E. coli host, by procedures well known in the art, such that amplification of the total DNA is effected, such that large quantities of covalently closed circular DNA for the subsequent transformation of a Bacillus host can be prepared from the transformed E. coli cells.

## Transformation Procedures

Procedures for the transformation of Bacillus cells, eg. B.t. kurstaki, are already known such as described in U.K. Patent Application 2199044A published 29 June 1988. Electroporation has been shown to increase transformation efficiency in E. coli to greater than $10^9$ colonies per ug of DNA, and has been reported to work for a wide variety of cell types and cell lines, including B. subtilis and B. cereus although at lower efficiencies. The improved process described herein allows transformation of Bacillus thuringiensis at a high efficiency, eg. an order of at least $10^6$ transformants per ug of DNA.

Cell types suitable for transformation by process of this invention include cry minus types such as the known B.t.k. cry 3B cells which have no plasmids and wild type Bacillus cells such as the native B.t. cells which carry endotoxin producing plasmids. As is well known in the art, Bacillus cells characteristically produce endotoxin in desired amounts only at their sporulation stage, and hence are grown to such stage in order best to obtain the products useful as insecticides. Hence, the plasmids containing DNA encoding a DET may be incorporated into Bacillus cells which are either devoid of endotoxin producing plasmids or already contain one or more of such plasmids. Bacillus cells transformed with plasmids are grown up by standard techniques and the recovered spore/endotoxin biomass may be used directly as insecticides in a manner known to the art.

The electroporation and recovery of cells in accord with the invention is carried out with cells while in a hypertonic state. For such purposes, three basic steps are desirably carried out involving: a) growing the cells in a hypertonic aqueous medium; b) subjecting the cells to high voltage pulsed current in the presence of the desired exogenous DNA while maintaining the hypertonic status; and c) incubating the thus treated cells in hypertonic aqueous incubation medium for a time sufficient to obtain intact transformed cells. A typical incubation will involve isolating and resuspending the treated cells and growing for eg. 2 hours, to obtain intact cells (which are capable of normal growth), and to express antibiotic resistance genes.

The electrotransformation used in this invention is a process which may be carried out with a GENE PULSER™ electroporation apparatus (Bio-Rad Laboratories, Richmond, California), in a manner generally consistent with the operation of such apparatus. In electroporation a brief, high voltage pulse is passed through a suspension of cells and DNA to be transformed to effect the transformation. The general conditions for conducting electrotransformations with such apparatus are known. The voltage desired to be employed will generally vary with capacitance, and a capacitance setting of 3 $\mu$F may be used with the apparatus. Voltages useful in step b) of our process may vary from about 1500 to 3000, more suitably 1700 to 2700 and preferably 1900 to 2600, depending upon cell density. The total time of pulse charge is no more than a few milliseconds and automatically determined from the voltage/capacitance setting on the Gene Pulser[TM] apparatus and the resistance of the sample. Optical density (O.D.) of the cells at harvest time for electroporation may vary but will be usually $OD_{600}$ 0.1 to 0.8. While densities of 0.2 to 0.5 may be suitably employed, it has been found that a density of 0.55 to 0.8, particularly 0.6 to 0.75, may be very satisfactorily employed and combined with higher voltages, eg. 2200 to 3000, preferably 2300 to 2600 (or to 2500 which represents to unmodified limit of the apparatus above indicated). During electroporation itself, the cells (in tubes) are kept on ice, eg. at a temperature of $0°$ C. to $5°$ C. The amount of DNA used in the transformations is generally between 50 ng to 5 ug per 800 ul. of cells, more usually 100 ng. to 4 ug., and preferably 0.5 ug to 2.0 ug. per 800 ul. of cells.

In general, in steps a), b) and c), the desired hypertonic status may be achieved by any of a variety of compounds which do not pass the semi-permeable cell membrane and which are not metabolized by or toxic to the cells. The saccharides, particularly mono- and disaccharides, are quite suitable, preferably sucrose and lactose, more preferably sucrose. The concentration of saccharides, eg. sucrose, employed is suitably of the order of about 0.35 M saccaride per liter of aqueous media or higher, to provide concentrations which are essentially isotonic with respect to the cell cytoplasm. Such osmotic status is in general preferably obtained with a concentration of from 0.35 M to 0.55 M of saccharides per liter of media, more preferably 0.38 M to 0.52 M of saccharide, eg. sucrose, per liter. The hypertonic aqueous media should be essentially neutral, ie. have a pH of from 5 to 9, preferably 6 to 8, with buffers being generally used for such purpose. Other ingredients such as conventional nutrients and salts may be included in the hypertonic media.

Cells may be prepared for transformation in step a) by growing in conventional manner, eg. with aeration and a temperature which usually between $20°$ C. to $40°$ C., preferably $20°$ C. to $38°$ C. (eg. $37°$ C). Desirably, growth is effected in an appropriate hypertonic nutrient medium, such as Brain Heart Infusion (BHI) with 500 mM sucrose, to exponential phase as measured by O.D. 600 nm of 0.1 to 0.8, eg. 0.2 to 0.5 but more preferably to 0.6 to 0.8. Lysozyme may be added to the culture, but the process may be operated to produce very good results without lysozyme and especially when the higher cell concentrations are used. The cells are concentrated by centrifugation resuspended in ice cold hypertonic buffered solution (5mM Hepes PH 7.0, 0.5 M sucrose). The cells are concentrated further by repeated centrifugation/wash steps to a final concentration of the order of $10^8$-$10^9$ cells/ml. and stored on ice.

The amount of lysozyme, when employed, should be well less than that normally used for the preparation of protoplasts, e.g. an amount not in excess of about 500 micrograms per ml. of hypertonic media. Such amount (concentration) will of course depend on various factors such as the osmotic pressure of the medium, its temperature, the desired reaction time etc. In general a suitable lysozyme concentration is of 20 to 300 microgram, e.g. of 200 microgram per ml of hypertonic aqueous medium (which is substantially lower than the 2 to 15 mg per ml which would be normally required for protoplasting purposes). Adequate distribution of lysozyme in the cell culture medium is desirably maintained. The reaction time will i.a. depend on the concentration and the quality of the lysozyme solution employed. In particular, the optimum lysozyme reaction time prior to transformation may be determined by preliminary assay in which samples of B.t. cells in the hypertonic medium are treated with the same given amount of lysozyme and individual such samples subjected to the same amount of lysozyme are reacted for different times. Preferably, the lysozyme is added to its final concentration at a lower cell concentration of about $OD_{600}$ 0.2 to 0.3 and the lysozyme-treated cells are harvested after 30 minutes at an $OD_{600}$ of 0.4 to 0.5 for use in electrotransformation.

In the electroporation step b), above, aliquots of buffered cells (eg. 800 ul) are mixed with the DNA, eg. vectors, to be transformed into the cells, and the suspension subjected to the pulsing at the desired voltage and capacitance settings until the apparatus signals discharge (audible beep with Gene Pulser[T]M).

Suitable DNA to be transformed comprises a DNA gene sequence operable in B.t. and encoding an endotoxin protein, an origin of replication in B.t. and a DNA gene sequence operable in B.t. and encoding antibiotic resistance. The electroporation of this invention works particularly well with B.t. kurstaki cells, B.t. tenebrionis cells and B.t. aisawa cells.

Following electrotransformation, the suspension comprising the transformed cells is then worked up in Step c), above, employing conventional methods but while securing the hypertonic status of the cells (when in solution/suspension). Thus the suspension is for example diluted in BHI/0.5M hypertonic medium and incubated. The suspension is incubated at a temperature of 20 to $40°$ C, e.g. at $37°$. The suspension is conveniently gently aerated (150 rpm) employing e.g. a shaking water bath. The required incubation is relatively short and generally need be carried out only for a time sufficient to allow expression of an antibiotic resistance gene carried by the exogenous DNA for the antibiotic resistance to be used to identify the transformed cells, or the expression of other marker genes incorporated for similar purposes. Such incubation also allows for whatever time may be needed for the treated cells to recover their ability to grow normally in Luria medium. Generally, the incubation is carried out for a period of at least about 60 minutes. An appropriate incubation time is no more than about 5 hours. Longer times may be employed but offer no particular advantage. Hence, incubation times are usually between 60 minutes to 5 hours, more preferably between 1.5 to 4 hours, e.g. 2 hours. After such incubation, the freshly prepared cells may be identified in a conventional manner and are capable of normal growth in Luria (normal) media similar to that of the untransformed parent cells.

Further, in accordance with the invention, transformation frequencies are further enhanced by transforming the B.t. cells with DNA which has been obtained from cells of the same subspecies type and character as the cells to be transformed. Such improvement, herein "homologous conditioning", may be due to minor adaptions, such as DNA methylation patterns, which particular cell types impart to heterologous DNA taken up by the cells, thereby making the DNA more subject to transformation back into the same cell type. In any event, when the DNA, eg. plasmids, vectors and the like, to be transformed into the ultimate B.t. host are constructed or amplified in a different host, it has been found desirable to transform the recovered DNA into cells of the same subspecies (preferably strain) to be ultimately or later transformed, recovering the DNA from said subspecies and then use the recovered DNA to again transform said subspecies, whereby distinctly higher frequencies of transformation may be obtained. When the ultimate host is a different strain (or mutant) of the same subspecies used as an intermediate host, similar type benefits may be obtained by transforming the ultimate host and collecting the resulting homologous conditioned DNA for again transforming the ultimate host cells. One advantage of such conditioning procedure is that DNA conditioned in a desired host can be mutagenized or otherwise altered after such conditioning and then transformed at distinctly high frequencies back into the same subspecies (or strain), thereby substantially improving mutagenisis procedures by avoiding less similar hosts and/or low frequency transformations as otherwise required to amplify the DNA or obtain the final product. Such conditioning may be used to advantage in mutagenizing by simply transforming a host, eg. a B.t., culturing, recovering the DNA, mutagenizing, and transforming the mutagenized DNA into a host of the same species, subspecies or strain, particularly the same subspecies.

It has also been found that the intermediate transformation of B.t. hosts capable of being transformed in good frequencies can be an important factor in enabling other, more difficult to transform B.t. hosts to be satisfactorily transformed, particularly with larger vectors carrying a B.t. endotoxin gene and/or DNA adapted for desired functioning or expression in different hosts, e.g. so-called shuttle vectors. For example, B.t. aizawai has been found very difficult to transform, particularly with the larger vectors. Difficulties have also been encountered with B.t. tenebrionis (also B.t. San Diego). For example, such DNA vectors when amplified and recovered from E. coli could not be transformed in our experience into B.t. aizawai or B.t. tenebrionis with sufficient frequency to enable the identification of positive isolates. However, when the E. coli derived DNA was first transformed into B.t. kurstaki, other DNA recovered from B.t.K. could then be transformed into B.t. tenebrionis with high frequency. However, the same DNA recovered from B.t. kurstaki could not be successfully transformed into B.t. aizawai but it was found that the same DNA recovered from B.t. tenebrionis could be transformed into B.t. aizawai in high frequency. Hence, the invention also provides a process for transforming B.t. cell hosts, particularly the more difficult to transform hosts such as tenebrionis and aizawai, with DNA amplified in E. coli, said process comprising transforming at least one intermediate, B.t. host which is a different subspecies or strain than the ultimate host, and transforming the ultimate B.t. host with the DNA recovered from the last to be transformed intermediate host. In another aspect, DNA recovered from B.t. tenebrionis is directly used to transform B.t. aizawai. In another aspect, DNA recovered from a B.t. kurstaki is directly used to transform B.t. tenebrionis. In general, DNA more easily transformed into one B.t. host than other B.t. subspecies or strain host may be transformed into the easier transformed host to condition the DNA prior to transforming the more difficult to transform B.t. host. Such conditioning transformations are particularly applicable to larger plasmids containing heterologous DNA comprising DNA encoding a B.t. operable gene for a B.t. endotoxin-like protein and an origin of replication operable in B.t. Such plasmids are typically truncated or full length endotoxin genes along with other DNA such as origin of replication. The heterologous DNA may further comprise an anti-biotic resistance gene, and essentially two shuttle vectors which comprise at least two origins of replications to allow for replication in at least two different hosts, eg. E. coli and B.t., and which may contain other DNA for a second or additional hosts such as promoter, RBS and other operator sequences and/or a second gene for another antibiotic resistance operable in a second or additional host. In general, the conditioning is effected simply by transforming a host, allowing for recovery as necessary to provide intact cells and culturing the cells to any volume desired or practical to recover the DNA for the subsequent or ultimate transformation.

This invention therefore further provides a process for transforming Bacillus thuringiensis (B.t.) host cells with DNA amplified in bacterial cells which are of a different species, subspecies or strain than such host cells, the improvement in increasing the frequency of such transformation comprising the steps of:

1) recovering the DNA transformed into such cells which are of a different species, subspecies or strain;

2) transforming the recovered DNA into B.t. cells of the same subspecies or strain as the host cells;

3) recovering, the DNA transformed in Step 2; and 4) transforming the host cells with the DNA recovered in Step 3 or a mutant of such DNA.

Such DNA may also be conditioned for transformation into more difficult to transform B.t. hosts or at high frequencies into B.t. hosts by transforming, constructing and/or amplifying the DNA in certain E. coli hosts such as those identified as GM31 and GM2163, both of which are available from New England Biolabs, Beverly, Massachusetts. We have, for example, successfully transformed both B.t. tenebrionis and B.t. aizawai with endotoxin-gene carrying shuttle vectors of the type described after amplifying the vectors in GM2163.

EXAMPLE 1: Preparation of plasmid pUC:BiB

A. Several micrograms of pBC16.1 DNA were digested with EcoR1 endonuclease and the linearized DNA gel isolated away from any remaining undigested plasmid DNA and/or contaminating chromosomal DNA. An aliquot of this purified 2.9kb EcoR1 fragment was ligated into DNA prepared the same way by digesting pUC18 with EcoR1. A control reaction of self-ligated pUC18 EcoR1 linearized DNA was used. Aliquots from these two ligations were used to transform competent E. coli host JM105. Cells were spread onto Yeast/Tryptone (YT) agar plates containing ampicillin, IPTG (isopropylthiogalactoside) and XGal. The IPTG/XGal selection allows for a colorimetric screening of insert containing pUC18 plasmids (white) due to the interruption of the Lac Z gene in the multiple cloning site of pUC plasmids. The control plates (no insert pBG16.1 DNA) showed less than 1% white colonies (background level). The experimental plates (pUC + pBC16.1 ligation) showed 20% white colonies.

Six white colonies were picked from the experimental transformation plate and used to inoculate YT liquid media containing 50 ug/ml ampicillin and grown overnight at 37 degrees celcius with shaking. Plasmid DNA mini-preparations were done on the six cultures and the DNA was analyzed by digestion with EcoR1. The pattern for all 6 clones indicated the presence of a 2.7kb pUC18 band and a 2.9 kb pBC16.1 band. Further analysis with other restriction enzymes indicated both orientations of the inserted pBC16.1 plasmid had been obtained, as expected. These clones were named pUC:B 1 through 6.

B. To evaluate whether pUC:B DNA would replicate, autonomously in B.t., a large scale plasmid preparation of pUC:B was done and purified on a cesium chloride gradient. This DNA was used to transform B.t. cryB cells by electroporation as described by this invention report. Tetracycline resistant colonies were obtained and plasmid DNA was, isolated. Restriction enzyme analysis confirmed pUC:B DNA was unaltered and could be used to re-transform competent E. coli or B.t. cells.

C. Formation of pUC:BiB: pUC:B was subjected to a partial digestion with EcoR1 and the overhangs thus produced on the linear molecule were filled in with DNA polymerase 1 Klenow fragment and dNTP's. The Bam H1 linkers shown below were purchased from New England Biolabs for ligation with the filled-in vector ends.

$$5'\text{CGGGATCCCG}3' \qquad \textbf{Bam H1 10mer}$$
$$3'\text{GCCCTAGGGC}5'$$

The BamH1 linker was present in the ligation reaction at a 50 fold molar excess compared to the pUC:B vector. Aliquots of this ligation were used to transform competent E. coli JM105 and clones were selected on YT agar plates containing 50 ug/ml ampicillin. Mini plasmid DNA prep. and restriction enzyme digestion confirmed the presence of the inserted linker at the previous EcoR1 site in 15% of the clones examined. The new plasmid containing this inserted BamH1 site is named pUC:BiB and is shown in Figure 1.

EXAMPLE 2: Preparation of plasmid pBEV210-TL

A. Plasmid pk8-1 was digested with Hpal and Sstl and the 1.5 kb fragment to serve as an insert in a subsequent ligation reaction was gel isolated and purified. Plasmid prAK was digested with Nrul and Sstl to isolate a 3.9 kb prAK fragment to serve as a vector. Equimolar amounts (0.5 pmole) of the 1.5kb

HpaI/SstI pk8-1 fragment and the 3.9kb NruI/SstI fragment were ligated together in a 20 ul reaction volume. 4 ul of this experimental ligation was used to transform competent E. coli strain JM105 and colonies were selected on YT/amp. plates. The number of transformants on the experimental plate was approximately 100 fold greater than control experiments with vector religated in the absence of the pk8-1 insert.

Six colonies from the experimental plate were grown in liquid culture for DNA preparation and restriction enzyme analysis. NdeI restriction enzyme was used and 6 out of 6 clones examined had the correct pattern (three fragments, 4211 bp, 737 bp and 409 bp). This clone is referred to as pBEV100.

B. Formation of plasmid pBEV210: 5-10 ug of pBEV-100 and pBT210 DNA were each digested with HpaI and SstI. The 1.6 kb HpaI/SstI fragment from pBEV100 (insert) and the 6.9 kb HpaI/SstI fragment of pBT210 (vector) were gel isolated and purified by standard techniques. The fragments were ligated together using a 4:1 molar ratio of insert to vector. E. coli strain JM105 competent cells were transformed with this ligation and colonies were selected on 20 ug/ml Chloramphenicol plates. Fourteen of the colonies were cultured from the experimental plate for DNA mini-prep. and restriction enzyme analysis. All 14 clones were shown to have the correct banding pattern when digested with HpaI and Sst 1 (6.9 + 1.6 kb). One of these colonies was cultured for a large scale plasmid preparation and was called pBEV210, and is illustrated in Figure 2.

C. To insert the B.t. DET 3' transcription termination loop into our pBEV210 clone, complementary oligonucleotides containing this DNA sequence as well as PvuI ends for cloning were designed and purchased from Research Genetics, Inc.

SMA I

5'   AAAACGGACATCACCTCCATTGAAACGGAGTGATGTCCGTTTTCCCGGGAT  3'

3' TATTTTGCCTGTAGTGGAGGTAACTTTGCCTCACTACAGGCAAAAGGGCCC   5'

PVU I "STICKY ENDS"

The oligonucleotides were first 5' phosphorylated with T4 polynucleotide kinase as described by Maniatis (1982 Cold Spring Harbour Laboratories, New York, MOLECULAR CLONING, A LABORATORY MANUAL). These oligonucleotides were annealed by placing them, in an equimolar ratio, in a heating block at 100 degrees celcius for five minutes. The block was then turned off and the temperature allowed to fall to 30 degrees celcius over a one and a half hour period, placed at room temperature for 5 minutes, and on ice for 5 minutes. Self-ligation of the annealled oligonucleotides was done and a ladder was visualized on a 2% agarose gel.

pBEV210 was prepared by partial PvuI digestion. 10-20 ug of pBEV210 was digested with PvuI for 5-10 minutes at 37 degrees celcius to obtain the linear DNA. This DNA was isolated on a 1% preparative agarose gel, eluted and purified according to standard procedures. The 8.6 kb linear pBEV210 vector was ligated with a 200 fold molar excess of the oligonucleotide cassette encoding the RNA transcription termination loop. JM105 competent E. coli cells were transformed with an aliquot of the ligation and the cells were plated onto YT/chloramphenicol for selection.

Thirteen colonies were selected from the experimental plate for DNA mini prep. and restriction enzyme analysis. Six of the 13 clones were shown to contain the inserted oligonucleotide based on the presence of the Sma 1 site internal to the oligo cassette. To check for the correct orientation of the inserted oligonucleotide, ScaI enzyme was used to digest these six clones along with SmaI. There is a ScaI site at the very 3' end of the DET gene in pBEV210. If the oligonucleotide was inserted in the proper orientation, a ScaI/SmaI digestion would give a 310 bp fragment. One of the six potential clones showed this fragment and it was designated pBEV210-TL (Figure 2).

EXAMPLE 3: Preparation of plasmid pBT1000

pBEV210-TL DNA was digested with the restriction enzymes HpaI and SmaI and the 4kb fragment thus produced was gel isolated and purified for use as an insert in a subsequent ligation. The vector pUC:BiB was digested with SmaI to produce a linear fragment of 5.6 kb. These two fragments were ligated with a five fold molar excess of insert to vector. Aliquots of this ligation mixture were used to transform competent E. coli strain JM105 and colonies were selected on YT/agar plates containing 50 ug/ml ampicillin.

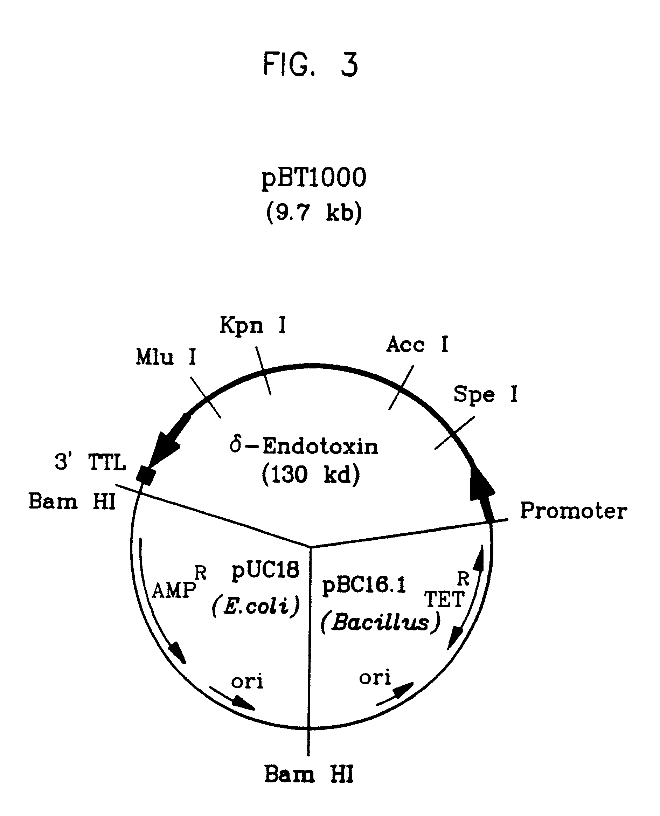

Transformants were replica plated onto YT/amp plates and colony lifts onto nitrocellulose membranes

13

were done. The clones were screened for the presence of the DET gene by hybridization with a 32-P radiolabelled Spel/Mlul DNA fragment of the DET gene. Positively hybridizing clones were further analyzed by DNA mini preps and restriction enzyme digestion. The results indicated that positive clones had been produced with the DET gene inserted into the PUC:BiB vector in both orientations as expected. The resulting clone, pBT1000 had the desired orientation and is shown in Figure 3.

EXAMPLE 4: Preparation of B.t.k. up-mutant plasmids

Ligations were performed between the 7Kb Mlul-Spel fragment of pBt1000 (gel isolated and purified) and the following mutation containing DNA fragments isolated from mutant DET clones (see also USSN 160,233 for mutant identifications):

   a) 2.6 Kb Mlul-Spel fragment of "C" in pBT210 (forms pBT 1001)
   b) 2.6 Kb Mlul-Spel fragment of "26-3" in pBT210 (forms pBT 1002)
   c) 2.6 Kb Mlul-Spel fragment of "36a65" in pBT210 (forms pBT 1003)
   d) 2.6 Kb Mlul-Spel fragment of "S" in pBT210 (forms pBT 1004)
   e) 2.6 Kb Mlul-Spel fragment of "98cl" in pBT210 (forms pBT 1005)

The ligations were transformed into E. coli JM105 and, in all cases, the transformation numbers were at least 10 fold higher for vector and insert than for religated vector alone. Transformants were screened by DNA mini-preps and Mlul-Spel restriction enzyme analysis and the presence of the inserts confirmed. DNA sequence analysis confirmed the correct point mutations were present for a positive clone from each of the five ligations listed above.

EXAMPLE 5: Transformation of Bacillus thuringiensis

A GENE PULSER™ transfection apparatus was purchased from Bio-Rad Laboratories. Reports in the literature on transformation of other cell types showed maximal efficiency occurred at a cell survival rate of approximately 50%. We selected this survival rate for our initial attempt at transformation and set up the following experiment to determine which voltage setting would give us this level of viability. Initial procedures used were those known to be effective for transformation of Bacillus subtilis.

A 100 ml culture of B.t. cry B was grown in Brain Heart Infusion (BHI) media purchased from Difco Laboratories to an optical density of 0.5 measured at 600 nm. Cells were sequentially pelleted by centrifugation at 4000 rpm for 10 minutes and resuspended in equal volume, 50% volume, 25% volume, and 12.5% volume of 10 mM ice cold Hepes buffer pH7.0. In the end, the cells have been concentrated 8 fold and are in 10mM Hepes buffer pH7.0 Aliquots containing 800 ul of this cell suspension were transferred into special sterile cuvettes supplied by the manufacturer. A cuvette containing these cells was then inserted into the holder and pulsed at one of the selected voltages, with the capacitance setting remaining constant at 3uF. Voltage settings used to generate a cell survival curve were 1300V, 1500V, 1700V, 1900V, 2100V and 2300V.

After pulsing, the cells were serially diluted by a factor of $10^5$ in sterile BHI media. Aliquots of the final dilution were plated onto YT/agar and incubated at 37°C for 10 hours. A control aliquot of cells which were diluted in the same manner but not pulsed was used to calculate the 100% survival value.

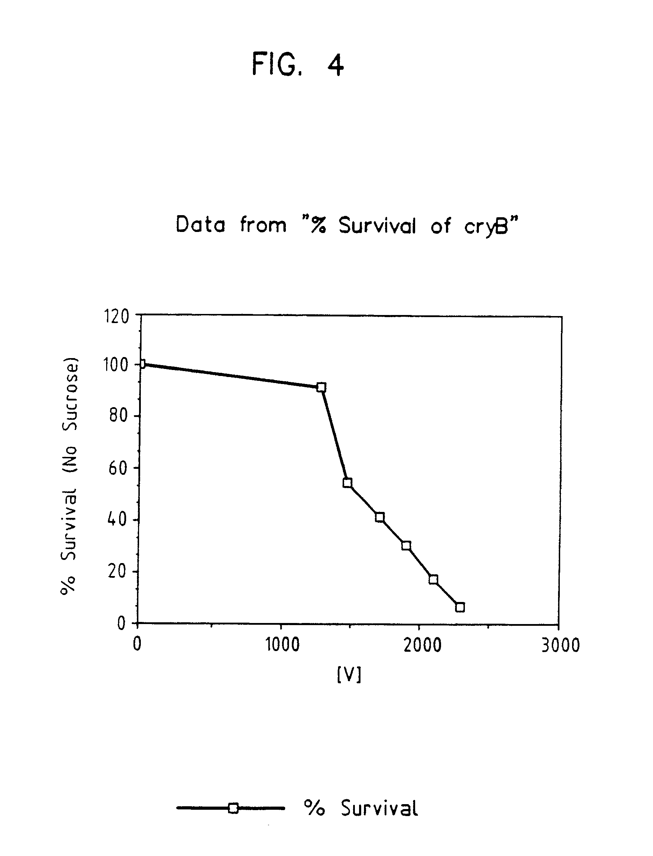

The number of colonies surviving each experimental voltage setting was divided by the number on the control plate to calculate percent survival. All settings were done in duplicate. The results indicated that a 50% survival was seen at approximately 1950 volts, as shown in Figure 4. Using this as our starting point, Cry B cells were prepared as described above and pulsed with 5 μg of pUCBiB DNA. After pulsing, cells were transferred into 10 mls sterile BHI media in 50 ml conical tubes and incubated at 37°C for 2 hours to allow for recovery and expression of tetracycline resistance. Following recovery, the cells were concentrated by centrifugation at 4000 rpm for 10 minutes. The pellet was resuspended in 500 μl YT media and plated onto two YT/agar plates containing 20-40 μg/ml tetracycline and incubated at 37°C overnight. A control experiment (minus DNA) was carried out in parallel.

Efficiencies of approximately 100 tetracycline resistant colonies were obtained per μg of DNA, demonstrating the efficacy of this approach with B. thuringiensis cells. Mini plasmid DNA preparations were done according to standard procedures. The isolated DNA was analyzed by restriction enzyme digestions, and both the identity and integrity of the plasmid DNA were confirmed. We subsequently attempted to transform cry B cells with pBT1000 DNA using these same conditions. We were able to obtain positive clones, although at ten-fold lower frequencies than our results with the vector pUCBiB DNA. The predicted patterns from various digestions of mini-prep DNA from positive cry B transformants confirmed both the presence and integrity of pBT1000 DNA.

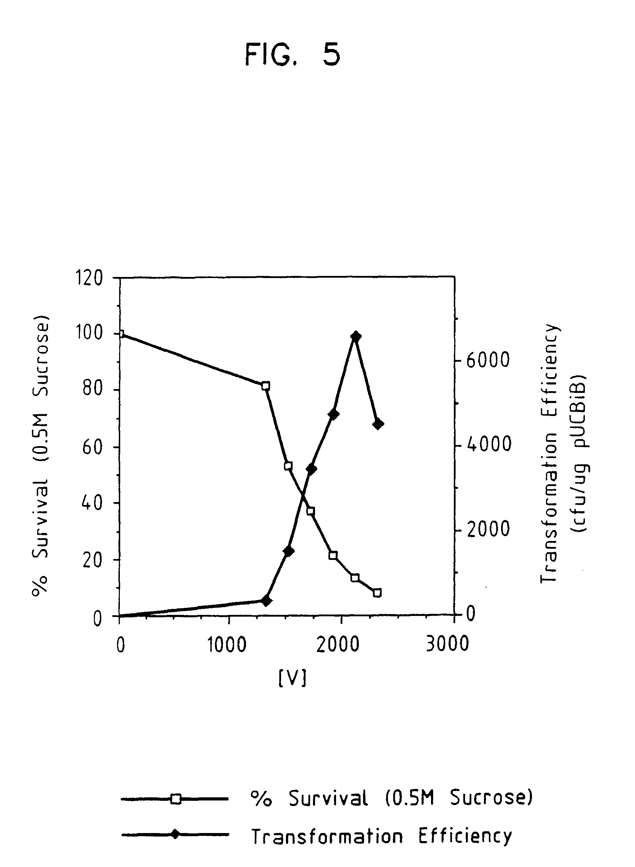

We wanted to directly examine percent cell survival versus efficiency of transformation to see if in fact 50% survival is optimal. Cells were prepared as described above and plated onto selective and non-selective media. The number of colonies from a control aliquot (non-pulsed, plated on non selective media) was taken as the 100% survival value. Colonies on YT media from samples pulsed at various voltages were counted to calculate % survival. Likewise, colonies on tetracycline-containing plates were counted to determine transformation efficiency. The relationship between cell survival and transformation efficiency is shown in Figure 5, for cells grown to 0D600 of about 0.4.

To analyze expression of DET in pBT1000 cryB transformed strains, (cry 1000), a 30 $\mu$l aliquot from an 18 hour culture was mixed with one quarter volume of sample buffer, heated at 100°C for 10 minutes and electrophoresed on a 9% SDS polyacrylamide gel. Control lanes of pUCBiB-transformed cells as well as molecular weight standards were included. Following electrophoresis, proteins were visualized by staining with Coomassie. Western blot analysis confirmed that the 130 Kd protein was immunoreactive to DET polyclonal antisera. Using gel scanning densitometry measurements, the level of DET expression in cry1000 strains was, estimated to be 30% of the total cellular protein when culture conditions were optimized.

EXAMPLE 6:    Highly efficient transformation of B.t. using improved electrotransformation procedures. Several approaches were taken to improve the efficiency of B.T. electrotransformation.

EXAMPLE 6A:

Lysozyme/Sucrose

Shown below are results we obtained after modifying the procedure to include lysozyme and sucrose. Differences between the previous procedure of Example 5 and a procedure involving lysozyme and sucrose are indicated below.

| Parameter | Previous | Current Lysozyme/Sucrose |
|---|---|---|
| Media | BHl | BHl/0.5 M sucrose |
| Temp. | 37°C | same |
| O.D. @ Harvest | 0.4-0.5 | same |
| Lysozyme | none | 200 µg/ml, 30 min. |
| Washes/resus-pension for transformation | 10 mM Hepes pH 7.0 | 5 mM Hepes pH 7.0, 0.5 M sucrose |
| Pulse | 1950V/3 µF Cap. | 2000 V/3 µF Cap. |
| Recovery | 3 hrs. in BHl | 2 hrs. BHl/0.5 M suc. |
| Selection | 20 µg/ml TET followed by 50 µg/ml | same |

Electrotransformation of cryB Bacillus thuringiensis cells using the previous conditions gave efficiencies of $10^2$ colonies per $\mu$g of pUCBib DNA. By modifying the procedure to include gentle lysozyme treatment in hypertonic growth media, we were able to get repeatable efficiencies of $10^4$ colonies per ug pUCBiB and $10^3$ per ug for pBT1000 when these plasmids were prepared from E. coli hosts, or a 100 fold enhancement over our initial results.

EXAMPLE 6B:

Effect of DNA Source

A crystal plus Javelin[R] substrain of B.t. kurstaki H.D. 562, herein also "SA11", was transformed with pBT1000 as described above for cryB to form strain SA1000. Similar transformation efficiencies were seen with this strain as were obtained for B.t. cryB ($10^4$ per ug of DNA for pUCBiB and $10^3$ per ug for pBT1000).

We examined what effect the source of DNA had on determining our efficiencies. Two strains of B.t., cry B and SA11, were transformed with DNA isolated from B.t. cry B, B.t. SA11, and E. coli JM105. In all cases, the lysozyme/sucrose method was used as described above. DNA concentration and extent of supercoiling were controlled for and the only variable in these experiments was the host cells from which the DNA was prepared. Results from reciprocal experiments (some done in duplicate) for transformations with pBT1000 DNA are given below:

| Strain Transformed | DNA Source | # of CFU's |
|---|---|---|
| Cry B | SA11 | $1.4 \times 10^3$, $1.8 \times 10^3$ |
| Cry B | Cry B | $1.5 \times 10^5$, $1.9 \times 10^5$ |
| Cry B | E. coli | $2.5 \times 10^3$ |
| SA11 | Cry B | $8.4 \times 10^3$ |
| SA11 | SA11 | $1.3 \times 10^5$ |
| SA11 | E. coli | $1.3 \times 10^4$ |

The results shown above indicate a significant preference for homologous DNA in B.t. transformations. This effect is likely due to differing patterns of DNA methylation recognized by each host.

EXAMPLE 6C:

High Efficiencies with modified Transformation Proceudre

A procedure carried out similar to that of Examples 5 and 6A obtained high efficiencies by growing cells to a higher density, and without using lysozyme. Differences between this modified procedure and the lysozyme/sucrose procedure are shown below.

| Parameter | Lysozyme/Sucrose (Example 6A) | Higher OD without Lysozyme |
|---|---|---|
| Media | BH1/0.5M Sucrose | same |
| Temp. | 37°C | same |
| OD at Harvest | 0.4-0.5 | 0.66 |
| Lysozyme | 200µg/ml. 30 min. | none |
| Washes/ resuspension for trans- formation | 5mM Hepes pH 7.0 0.5 M Sucrose | same |
| Pulse | 2000V/3µF | 2500V/3µF |
| Recovery | 2 hrs. BH1/0.5M sucrose | same |
| Selection | 20 µg/ml TET followed by 50 µg/ml | same |

In this Example 6C it was found that growing the cells to a higher OD600, eg. 0.6 to 0.8, over the course of an additional one hour could improve efficiencies by as much as 50 fold using sucrose but without adding lysozyme. By this procedure it was found that SA11 cells could be transformed in efficiencies of $5 \times 10^5$ colonies per ug. of pUCBiB and $1 \times 10^5$ colonies per $\mu$g. of pBT1000.

EXAMPLE 7: Toxicity of transformed B.t. strains Cry B and SA11

The following toxicity data (evaluation for insecticidal activity) were obtained by growing the B.t. cells to be evaluated in Dulmage medium for 4 days at 30°C. The amount of toxin present in each culture was equivalent as judged by SDS PAGE and scanning gel densitometry. Five different quantities of each cultured cell system to be evaluated were mixed in cups with artificial diet to provide a range of concentrations of from 0.12% to 10% expressed as a volume percent B.t. culture present. One second instar larvae was placed in each cup and each concentration was run 10 times (total 50 cups per culture). Percent mortality versus percent concentration were graphed and the lowest dose giving fifty percent (50%) mortality ($LD_{50}$ value) was calculated for each culture to be evaluated. Relative toxicities to pBT 1000 were also calculated for the pBT1000 series (pBT1000 to pBT 1006). The tables below indicate the results obtained. The Heliothis evaluated was H. virescens and the spodoptera evaluated was littoralis.

Table 1 - pBt 1000 Series in Cry B - H. virescens and
Spodoptera littoralis

| Plasmid | H. Virescens Relative to pBT 1000(%) | S.littoralis Approximate $LD_{50}$ |
|---------|----------------|----------------|
| pBT 1000 | 100 | 16.1 |
| pBt 1001 | 95 | 10.0 |
| pBT 1002 | 180 | 5.6 |
| pBT 1003 | 165 | NA* |
| pBT 1004 | 120 | NA* |
| pBt 1005 | 105 | NA* |
| pBT 1006 | 50 | NA* |

NA: Not active at highest concentration tested.

Table 2 - pBT 1000 Series in SA11

| Plasmid | H. virescens Approximate $LD_{50}$ | Spodoptera littoralis Approximate $LD_{50}$ |
|---|---|---|
| pBt 1000 | 0.06 | 1.2 |
| pBT 1001 | 0.9 | 8.2 |
| pBT 1002 | 0.09 | 1.6 |
| pBT 1003 | 0.15 | 3.75 |
| pBT 1004 | 2.2 | 9.1 |
| pBT 1005 | 0.1 | 1.4 |
| pBT 1006 | 1.2 | 10.1 |
| SA11 - control | 0.13 | 3.8 |

Table 2A - Certain pBT1000 Series Candidates in SA11 against Trichoplusia Ni

| Plasmid | $LD_{50}$ | Relative to SA11 (%) |
|---|---|---|
| pBT1000 | 0.47 | 87 |
| pBT1005 | 0.245 | 167 |
| SA11 - Control | 0.41 | 100 |

Table 3 - Advanced Screening of pBT 1000 Series Candidates Relative to SA11

The following results were obtained after averaging the results of seven replications of the assay procedure described above.

19

| Plasmid in SA11 | H. virescens Relative to SA11 (%) | S. littoralis Relative to SA11 (%) |
|---|---|---|
| pBT1000 | ca 100 | 242 |
| pBT1002 | ca 100 | 313 |
| pBT1005 | 240 | 404 |
| SA11-Control | 100 | 100 |

The plasmid pBT1000 can be used to express hybrid DET genes. For example, the SpeI/KpnI fragment of a B.t. kurstaki DET gene of the cryI A(a) type (Hermon Hofte and H. R. Whiteley, Microbiological Reviews, June 1989, p. 242-55. Insecticidal Crystal Proteins of B.t.) contained in pES-1 (U.S. Patent No. 4,467,036. Schnepf et al. August 21, 1984.) can be inserted into pBT1000 digested with these same restriction enzymes. All of the amino acid changes within the active portion of the toxin genes contained in plasmids pES-1 and pBT1000 fall within a fragment defined by the restrictions enzymes SpeI and KpnI in common to both genes. The amino acids upstream of SpeI are identical in both genes and those downstream of KpnI are in the coding region of the inactive C-terminal portion of the protoxin. Our aim was to replace the SpeI/KpnI fragment of the DET gene in our vector, pBT1000, with the corresponding fragment from the pES-1. The resulting clone has all of the DNA coding for the active portion of the DET from the cryI A(a) gene and a C-terminal portion of the DET gene from the cryI A(b) gene of B.t.w. Construction of this hybrid DET gene for expression in Bacillus and E. coli hosts is described in more detail in the following example.

EXAMPLE 8: Preparation of pBT 2000

Due to the occurrence of KpnI sites elsewhere in the expression plasmid, we first cloned this hybrid gene into the vector pBEV210TL, as an intermediate step. Positive clones were then used to isolate the resulting hybrid DET gene for ligation into the B.t. expression vector. Details of these experiments are as follows:

The SpeI/KpnI 6.6 kb vector fragment from pBEV210TL and the SpeI/KpnI 2.0 kb insert fragment from pES-1 were prepared from the indicated plasmids by digesting 5 ug of each of pES-1 and pBEV210TL with 50 units of SpeI and KpnI for 3 hours at 37° C. Preparative agarose gels were run and the fragments were isolated and purified by DEAE disposable columns (Elutip). Ligations were set up with 0.06 picomoles (pmoles) of insert DNA and 0.02 pmoles of vector in a 20 microliter (ul) reaction volume. A control ligation was also set up containing 0.02 pmoles of vector in the same final volume. A 5 ul aliquot from each ligation was used to transform competent E. coli strain JM105 cells and transformed cells were selected on chloramphenicol plates (20 ug/ml). The ratio of colonies from the experimental vs. control ligation was greater than 50 to 1. Individual colonies from the experimental ligation were grown in liquid media with chloramphenicol (20ug/ml) for mini plasmid DNA preparation.

Plasmid DNA from twelve different isolates was prepared and analyzed by digestion with Pvu II. The kurstaki gene in pES-1 has a Pvu II site in the SpeI/KpnI fragment and lacks an Acc I site present in this region of the B.t.w. cloned DET gene. These differences distinguish between these highly conserved genes. Analysis of the fragment sizes produced with these two enzymes confirmed the desired hybrid DET clone, herein pBEV2000.

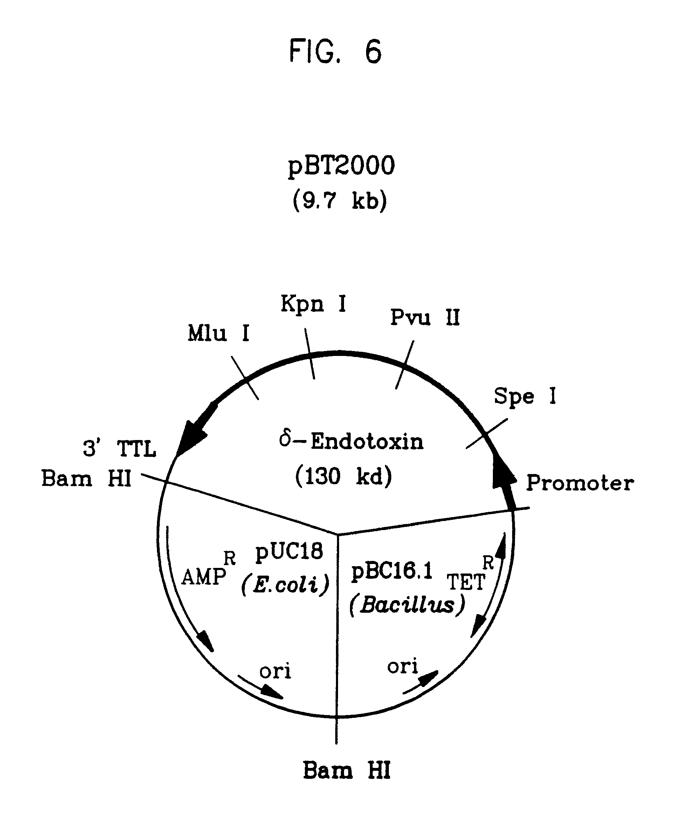

A large scale DNA preparation of pBEV2000 DNA was done to isolate a fragment containing the hybrid DET gene for cloning into our expression vector. The SpeI/MluI 7 kb vector fragment from pBT1000 and the SpeI/MluI 2.6 kb insert fragment from pBEV2000 were prepared, isolated and ligated as described above for pBEV2000. Plasmid DNA from resulting clones were analyzed by digestion with ACC I and PvuII enzymes. All six clones were confirmed as having the pES-1 gene up to codon 723 followed by the C-terminus from our 5.3kb type gene and all regulatory (5' and 3') sequences present in our pBT1000 expression vector. This new clone was denominated pBT2000 and is shown in Figure 6.

Initial attempts to transform B.t. tenebrionis with DNA prepared from E. coli were unsuccessful. High

efficiencies ($10^5$ per ug DNA) were obtained when the DNA was first amplified in our transformed B.t. kurstaki strain SA1000. The resulting hybrid strain is referred to as TEN 1000. Likewise we were unable to isolate positive transformants of B.t. aizawai using pBT1000 DNA isolated from E. coli JM105 or strain SA1000, but B.t. aizawai could be transformed at a high efficiency when we isolated pBT1000 DNA from our Ten.1000 strain. In summary, we have discovered highly specific restriction/modification systems of different strains of B.t. which can be used to permit high efficiency transformation of crystal plus strains of B.t. (e.g. B.t. kurstaki, B.t. aizawai, B.t. tenebrionis). We have used this technology to produce the following hybrid strains:

SA1000 B:t. kurstaki strain transformed with pBT1000 [plasmid coding for the CryI(A)b gene of B.t. wuhanensis (ref. E. coli mutant Patent Appl.].

Ten 1000 B.t. tenebrionis strain transformed with pBT1000 DNA.

Aiz 1000 B.t. aizawai strain transformed with pBT1000 DNA.

EXAMPLE 9: Assay of B.t. aizawai

B.t. aizawai (strain HD-137) transformed with pBT 1000 as indicated above was evaluated against H. virescens and spodoptera in a comparison against the native B.t. aizawai strain with the result that the transformed species (herein A12 1000) showed an $LD_{50}$ against the Heliothis of about 5.5 and against Spodoptera of about 18 while the wild type showed $LD_{50}$ values of about 3 and 4.5 respectively.

EXAMPLE 10: Assay of B.t. tenebrionis

B.t. tenebrionis transformed with pBT 1000 as indicated above (to produce TEN 1000) was evaluated against H. virescens and Phaedon (cochleria) in a comparison the native B.t. tenebrionis strain with the result that the native strain showed no practical affect against the Heliothis and the TEN 1000 showed 50% of the activity of the native strain against Phaedon but also showed an $LD_{50}$ of 8.39 against the Heliothis. In this example, the assays for Phaedon toxicity was a leaf disc assay in which the culture to be evaluated was sprayed directly onto leaf discs and allowed to dry. Ten insects ($2^{nd}$ instar) were then allowed to feed on the leaf discs for 7 days and toxicity then scored as percent mortality.

EXAMPLE 11:

The plasmid pBT2000 was amplified in E. coli JM105, the DNA recovered and transformed by electroporotim as above described (using hypertonic media procedure) into B.t.k. SA11, the DNA recovered and transformed by electroporation as above described (using hypertonic media procedure) into B.t. tenebrionis to obtain transformed cell herein identified as TEN 2000. Plasmid DNA (pBT2000) was then recovered from TEN 2000 and transformed into B.t. aizawai (HD-137) by electroporation as above described (using hypertonic media procedure) to obtain transformed cells herein identified as AIZ 2000.

EXAMPLE 12:

The transforants TEN 2000 and AIZ 2000 were evaluated for toxicity (unsectidal activity) by the assays above described with the following results.

A) B.t. tenebrionis cells

| Strain B.t. tenebrionis | Phaedon (% mortality) | Heliothis $(LD_{50})$ |
|---|---|---|
| TEN 1000 | 100 | 0 |
| TEN 2000 | 50 | 8.39 |
| TEN 2000 | 90 | 8.0 |
| Cry B/pBT 1000 (control) | 10 (background) | 8.07 |
| Cry B/pBT 2000 (control0 | 10 (background) | 11.56 |

B) B.t. aizawai cells

| Strain | Heliothis $(LD_{50})$ | Spodoptera $(LD_{50})$ |
|---|---|---|
| B.t. aizawai (HD-137) | 3 | 4.5 |
| AIZ 1000 | 5.5 | 18 |
| AIZ 2000 | 1 | 2.2 |

The above data indicates that AIZ 2000 is a very potent new B.t. strain with excellent potency towards both Heliothis and Spodoptera insects, and indicates the desirability of transforming B.t. aizawai with a plasmid expressing an endotoxin having an active toxic portion substantially the same or identical to the active portion (first 610 amino acids) of the pES-1 endotoxin (the amino acid sequence of which is described in Schnepf, et al., J. Biol. chem. Vol. 260 (1985), pgs. 6264-6272). The active portion of the endotoxin has at least about 50 amino acid differences from the B.t.w. active sequence and it is indicated that many changes or mutations in the pES-1 sequence, eg. as many as at least a majority or more of the 50 or more that differ, may be made while retaining the unexpected advantage over the parent B.t. aizawai, ie. greater activity against H. virescens and exigua, and all such mutants are considered within the invention. The term "heterogenous" as used herein with reference to DNA and cells transformed therewith indicate that all or any portion of the sequence of the DNA is not native to or naturally found within the cells in question.

## TABLE A

```
    (a)                                                        -46
     GG ATC CGT TTT AAA TTG TAG TAA TGA AAA ACA GTA TTA
        Ile Arg Phe Lys Leu *** *** *** Lys Thr Val Leu


TAT CAT AAT GAA TTG GTA TCT TAA TAA AAG AGA TGG AGG TAA CTT
Tyr His Asn Glu Leu Val Ser *** *** Lys Arg Trp Arg *** Leu
(-15)
                                                              45
ATG GAT AAC AAT CCG AAC ATC AAT GAA TGC ATT CCT TAT AAT TGT
Met Asp Asn Asn Pro Asn Ile Asn Glu Cys Ile Pro Tyr Asn Cys
(1)         (4)                                           (15)


TTA AGT AAC CCT GAA GTA GAA GTA TTA GGT GGA GAA AGA ATA GAA
Leu Ser Asn Pro Glu Val Glu Val Leu Gly Gly Glu Arg Ile Glu


ACT GGT TAC ACC CCA ATC GAT ATT TCC TTG TCG CTA ACG CAA TTT
Thr Gly Tyr Thr Pro Ile Asp Ile Ser Leu Ser Leu Thr Gln Phe

                                                          (b)
CTT TTG AGT GAA TTT GTT CCC GGT GCT GGA TTT GTG TTA GGA CTA
Leu Leu Ser Glu Phe Val Pro Gly Ala Gly Phe Val Leu Gly Leu
                                                          (60)


GTT GAT ATA ATA TGG GGA ATT TTT GGT CCC TCT CAA TGG GAC GCA
Val Asp Ile Ile Trp Gly Ile Phe Gly Pro Ser Gln Trp Asp Ala

                                                  .       n-1
TTT CTT GTA CAA ATT GAA CAG TTA ATT AAC CAA AGA ATA GAA GAA
Phe Leu Val Gln Ile Glu Gln Leu Ile Asn Gln Arg Ile Glu Glu
                                                          (m-1)


             (c)    300
TTC GCT AGG AAC CAA GCC ATT TCT AGA TTA GAA GGA CTA AGC AAT
Phe Ala Arg Asn Gln Ala Ile Ser Arg Leu Glu Gly Leu Ser Asn
                                            (100)


CTT TAT CAA ATT TAC GCA GAA TCT TTT AGA GAG TGG GAA GCA GAT
Leu Tyr Gln Ile Tyr Ala Glu Ser Phe Arg Glu Trp Glu Ala Asp


CCT ACT AAT CCA GCA TTA AGA GAA GAG ATG CGT ATT CAA TTC AAT
Pro Thr Asn Pro Ala Leu Arg Glu Glu Met Arg Ile Gln Phe Asn
                                                          (135)
```

Note: (a) is Bam HI site
      (b) is Spe I site
      (c) is Xba I site

```
GAC ATG AAC AGT GCC CTT ACA ACC GCT ATT CCT CTT TTT GCA GTT
Asp Met Asn Ser Ala Leu Thr Thr Ala Ile Pro Leu Phe Ala Val
        (140)

                                                        495
CAA AAT TAT CAA GTT CCT CTT TTA TCA GTA TAT GTT CAA GCT GCA
Gln Asn Tyr Gln Val Pro Leu Leu Ser Val Tyr Val Gln Ala Ala
                                                        (165)

                        523
AAT TTA CAT TTA TCA GTT TTG AGA GAT GTT TCA GTG TTT GGA CAA
Asn Leu His Leu Ser Val Leu Arg Asp Val Ser Val Phe Gly Gln
                                                        (180)

        549                             577
AGG TGG GGA TTT GAT GCC GCG ACT ATC AAT AGT CGT TAT AAT GAT
Arg Trp Gly Phe Asp Ala Ala Thr Ile Asn Ser Arg Tyr Asn Asp
                                                        (195)

                        606       n-348       624
TTA ACT AGG CTT ATT GGC AAC TAT ACA GAT CAT GCT GTA CGC TGG
Leu Thr Arg Leu Ile Gly Asn Tyr Thr Asp His Ala Val Arg Trp
                                 (m-116)            (210)

                                                (d)   675
TAC AAT ACG GGA TTA GAG CGT GTA TGG GGA CCG GAT TCT AGA GAT
Tyr Asn Thr Gly Leu Glu Arg Val Trp Gly Pro Asp Ser Arg Asp
                                                        (225)

TGG ATA AGA TAT AAT CAA TTT AGA AGA GAA TTA ACA CTA ACT GTA
Trp Ile Arg Tyr Asn Gln Phe Arg Arg Glu Leu Thr Leu Thr Val

TTA GAT ATC GTT TCT CTA TTT CCG AAC TAT GAT AGT AGA ACG TAT
Leu Asp Ile Val Ser Leu Phe Pro Asn Tyr Asp Ser Arg Thr Tyr
                                                        (255)

CCA ATT CGA ACA GTT TCC CAA TTA ACA AGA GAA ATT TAT ACA AAC
Pro Ile Arg Thr Val Ser Gln Leu Thr Arg Glu Ile Tyr Thr Asn

CCA GTA TTA GAA AAT TTT GAT GGT AGT TTT CGA GGC TCG GCT CAG
Pro Val Leu Glu Asn Phe Asp Gly Ser Phe Arg Gly Ser Ala Gln

GGC ATA GAA GGA AGT ATT AGG AGT CCA CAT TTG ATG GAT ATA CTT
Gly Ile Glu Gly Ser Ile Arg Ser Pro His Leu Met Asp Ile Leu

AAC AGT ATA ACC ATC TAT ACG GAT GCT CAT AGA GGA GAA TAT TAT
Asn Ser Ile Thr Ile Tyr Thr Asp Ala His Arg Gly Glu Tyr Tyr

TGG TCA GGG CAT CAA ATA ATG GCT TCT CCT GTA GGG TTT TCG GGG
Trp Ser Gly His Gln Ile Met Ala Ser Pro Val Gly Phe Ser Gly
```

Note: (d) is Xba I site

```
CCA GAA TTC ACT TTT CCG CTA TAT GGA ACT ATG GGA AAT GCA GCT
Pro Glu Phe Thr Phe Pro Leu Tyr Gly Thr Met Gly Asn Ala Ala

CCA CAA CAA CGT ATT GTT GCT CAA CTA GGT CAG GGC GTG TAT AGA
Pro Gln Gln Arg Ile Val Ala Gln Leu Gly Gln Gly Val Tyr Arg

ACA TTA TCG TCC ACT TTA TAT AGA AGA CCT TTT AAT ATA GGG ATA
Thr Leu Ser Ser Thr Leu Tyr Arg Arg Pro Phe Asn Ile Gly Ile

AAT AAT CAA CAA CTA TCT GTT CTT GAC GGG ACA GAA TTT GCT TAT
Asn Asn Gln Gln Leu Ser Val Leu Asp Gly Thr Glu Phe Ala Tyr

GGA ACC TCC TCA AAT TTG CCA TCC GCT GTA TAC AGA AAA AGC GGA
Gly Thr Ser Ser Asn Leu Pro Ser Ala Val Tyr Arg Lys Ser Gly

ACG GTA GAT TCG CTG GAT GAA ATA CCG CCA CAG AAT AAC AGC GTG
Thr Val Asp Ser Leu Asp Glu Ile Pro Pro Gln Asn Asn Asn Val

CCA CCT AGG CAA GGA TTT AGT CAT CGA TTA AGC CAT GTT TCA ATG
Pro Pro Arg Gln Gly Phe Ser His Arg Leu Ser His Val Ser Met
                                                          1350
TTG CGT TCA GGC TTT AGT AAT AGT AGT GTA AGT ATA ATA AGA GCT
Phe Arg Ser Gly Phe Ser Asn Ser Ser Val Ser Ile Ile Arg Ala
                                                         (450)

CCT ATG TTC TCT TGG ATA CAT CGT AGT GCT GAA TTT AAT AAT ATA
Pro Met Phe Ser Trp Ile His Arg Ser Ala Glu Phe Asn Asn Ile

ATT CCT TCA TCA CAA ATT ACA CAA ATA CCT TTA ACA AAA TCT ACT
Ile Pro Ser Ser Gln Ile Thr Gln Ile Pro Leu Thr Lys Ser Thr

AAT CTT GGC TCT GGA ACT TCT GTC GTT AAA GGA CCA GGA TTT ACA
Asn Leu Gly Ser Gly Thr Ser Val Val Lys Gly Pro Gly Phe Thr

GGA GGA GAT ATT CTT CGA AGA ACT TCA CCT GGC CAG ATT TCA ACC
Gly Gly Asp Ile Leu Arg Arg Thr Ser Pro Gly Gln Ile Ser Thr

TTA AGA GTA AAT ATT ACT GCA CCA TTA TCA CAA AGA TAT CGG GTA
Leu Arg Val Asn Ile Thr Ala Pro Leu Ser Gln Arg Tyr Arg Val

AGA ATT CGC TAC GCT TCT ACC ACA AAT TTA CAA TTC CAT ACA TCA
Arg Ile Arg Tyr Ala Ser Thr Thr Asn Leu Gln Phe His Thr Ser

ATT GAC GGA AGA CCT ATT AAT CAG GGG AAT TTT TCA GCA ACT ATG
Ile Asp Gly Arg Pro Ile Asn Gln Gly Asn Phe Ser Ala Thr Met

AGT AGT GGG AGT AAT TTA CAG TCC GGA AGC TTT AGG ACT GTA GGT
Ser Ser Gly Ser Asn Leu Gln Ser Gly Ser Phe Arg Thr Val Gly

TTT ACT ACT CCG TTT AAC TTT TCA AAT GGA TCA AGT GTA TTT ACG
Phe Thr Thr Pro Phe Asn Phe Ser Asn Gly Ser Ser Val Phe Thr

TTA AGT GCT CAT GTC TTC AAT TCA GGC AAT GAA GTT TAT ATA GAT
Leu Ser Ala His Val Phe Asn Ser Gly Asn Glu Val Tyr Ile Asp
```

25

```
CGA ATT GAA TTT GTT CCG GCA GAA GTA ACC TTT GAG GCA GAA TAT
Arg Ile Glu Phe Val Pro Ala Glu Val Thr Phe Glu Ala Glu Tyr
                                          (610)

GAT TTA GAA AGA GCA CAA AAG GCG GTG AAT GAG CTG TTT ACT TCT
Asp Leu Glu Arg Ala Gln Lys Ala Val Asn Glu Leu Phe Thr Ser

TCC AAT CAA ATC GGG TTA AAA ACA GAT GTG ACG GAT TAT CAT ATT
Ser Asn Gln Ile Gly Leu Lys Thr Asp Val Thr Asp Tyr His Ile

GAT CAA GTA TCC AAT TTA GTT GAG TGT TTA TCT GAT GAA TTT TGT
Asp Gln Val Ser Asn Leu Val Glu Cys Leu Ser Asp Glu Phe Cys

CTG GAT GAA AAA AAA GAA TTG TCC GAG AAA GTC AAA CAT GCG AAG
Leu Asp Glu Lys Lys Glu Leu Ser Glu Lys Val Lys His Ala Lys

CGA CTT AGT GAT GAG CGG AAT TTA CTT CAA GAT CCA AAC TTT AGA
Arg Leu Ser Asp Glu Arg Asn Leu Leu Gln Asp Pro Asn Phe Arg

GGG ATC AAT AGA CAA CTA GAC CGT GGC TGG AGA GGA AGT ACG GAT
Gly Ile Asn Arg Gln Leu Asp Arg Gly Trp Arg Gly Ser Thr Asp

ATT ACC ATC CAA GGA GGC GAT GAC GTA TTC AAA GAG AAT TAC GTT
Ile Thr Ile Gln Gly Gly Asp Asp Val Phe Lys Glu Asn Tyr Val

                      (e)
ACG CTA TTG GGT ACC TTT GAT GAG TGC TAT CCA ACG TAT TTA TAT
Thr Leu Leu Gly Thr Phe Asp Glu Cys Tyr Pro Thr Tyr Leu Tyr
          (723)
                                                         2250
CAA AAA ATA GAT GAG TCG AAA TTA AAA GCC TAT ACC CGT TAC CAA
Gln Lys Ile Asp Glu Ser Lys Leu Lys Ala Tyr Thr Arg Tyr Gln
                                                        (750)

TTA AGA GGG TAT ATC GAA GAT AGT CAA GAC TTA GAA ATC TAT TTA
Leu Arg Gly Tyr Ile Glu Asp Ser Gln Asp Leu Glu Ile Tyr Leu

ATT CGC TAC AAT GCC AAA CAC GAA ACA GTA AAT GTG CCA GGT ACG
Ile Arg Tyr Asn Ala Lys His Glu Thr Val Asn Val Pro Gly Thr

GGT TCC TTA TGG CCG CTT TCA GCC CCA AGT CCA ATC GGA AAA TGT
Gly Ser Leu Trp Pro Leu Ser Ala Pro Ser Pro Ile Gly Lys Cys
```

Note: (e) is Kpn I site

26

```
GGA GAA CCG AAT CGA TGC GCA CCA CAA CTT GAA TGG AAT CCA GAT
Gly Glu Pro Asn Arg Cys Ala Pro Gln Leu Glu Trp Asn Pro Asp

CTA GAT TGT TCC TGC AGA GAC GGA GAA AAA TGT GCC CAT CAT TCC
Leu Asp Cys Ser Cys Arg Asp Gly Glu Lys Cys Ala His His Ser

CAT CAT TTC TCC TTG GAC ATT GAT GTT GGA TGT ACA GAC TTA AAT
His His Phe Ser Leu Asp Ile Asp Val Gly Cys Thr Asp Leu Asn
                                                          (840)

GAG GAC TTA GGT GTA TGG GTG ATA TTC AAG ATT AAG ACG CAA GAT
Glu Asp Leu Gly Val Trp Val Ile Phe Lys Ile Lys Thr Gln Asp

GGC CAT GCA AGA CTA GGA AAT CTA GAA TTT CTC GAA GAG AAA CCA
Gly His Ala Arg Leu Gly Asn Leu Glu Phe Leu Glu Glu Lys Pro

TTA GTA GGA GAA GCA CTA GCT CGT GTG AAA AGA GCG GAG AAA AAA
Leu Val Gly Glu Ala Leu Ala Arg Val Lys Arg Ala Glu Lys Lys

                                                          2700
TGG AGA GAC AAA CGT GAA AAA TTG GAA TGG GAA ACA AAT ATT GTT
Trp Arg Asp Lys Arg Glu Lys Leu Glu Trp Glu Thr Asn Ile Val
                                                          (900)

TAT AAA GAG GCA AAA GAA TCT GTA GAT GCT TTA TTT GTA AAC TCT
Tyr Lys Glu Ala Lys Glu Ser Val Asp Ala Leu Phe Val Asn Ser

CAA TAT GAT AGA TTA CAA GCG GAT ACC AAC ATC GCG ATG ATT CAT
Gln Tyr Asp Arg Leu Gln Ala Asp Thr Asn Ile Ala Met Ile His

GCG GCA GAT AAA CGC GTT CAT AGC ATT CGA GAA GCT TAT CTG CCT
Ala Ala Asp Lys Arg Val His Ser Ile Arg Glu Ala Tyr Leu Pro

GAG CTG TCT GTG ATT CCG GGT GTC AAT GCG GCT ATT TTT GAA GAA
Glu Leu Ser Val Ile Pro Gly Val Asn Ala Ala Ile Phe Glu Glu

TTA GAA GGG CGT ATT TTC ACT GCA TTC TCC CTA TAT GAT GCG AGA
Leu Glu Gly Arg Ile Phe Thr Ala Phe Ser Leu Tyr Asp Ala Arg

AAT GTC ATT AAA AAT GGT GAT TTT AAT AAT GGC TTA TCC TGC TGG
Asn Val Ile Lys Asn Gly Asp Phe Asn Asn Gly Leu Ser Cys Trp

AAC GTG AAA GGG CAT GTA GAT GTA GAA GAA CAA AAC AAC CAC CGT
Asn Val Lys Gly His Val Asp Val Glu Glu Gln Asn Asn His Arg

TCG GTC CTT GTT GTT CCG GAA TGG GAA GCA GAA GTG TCA CAA GAA
Ser Val Leu Val Val Pro Glu Trp Glu Ala Glu Val Ser Gln Glu

GTT CGT GTC TGT CCG GGT CGT GGC TAT ATC CTT CGT GTC ACA GCG
Val Arg Val Cys Pro Gly Arg Gly Tyr Ile Leu Arg Val Thr Ala

TAC AAG GAG GGA TAT GGA GAA GGT TGC GTA ACC ATT CAT GAG ATC
Tyr Lys Glu Gly Tyr Gly Glu Gly Cys Val Thr Ile His Glu Ile
                                                          (1050)
```

EP 0 433 945 A2

```
GAG AAC AAT ACA GAC GAA CTG AAG TTT AGC AAC TGT GTA GAA GAG
Glu Asn Asn Thr Asp Glu Leu Lys Phe Ser Asn Cys Val Glu Glu

                                                          3240
GAA GTA TAT CCA AAC AAC ACG GTA ACG TGT AAT GAT TAT ACT GCG
Glu Val Tyr Pro Asn Asn Thr Val Thr Cys Asn Asp Tyr Thr Ala
                                                        (1080)

ACT CAA GAA GAA TAT GAG GGT ACG TAC ACT TCT CGT AAT CGA GGA
Thr Gln Glu Glu Tyr Glu Gly Thr Tyr Thr Ser Arg Asn Arg Gly

TAT GAC GGA GCT TAT GAA AGC AAT TCT TCT GTA CCA GCT GAT TAT
Tyr Asp Gly Ala Tyr Glu Ser Asn Ser Ser Val Pro Ala Asp Tyr

GCA TCA GCC TAT GAA GAA AAA GCA TAT ACA GAT GGA CGA AGA GAC
Ala Ser Ala Tyr Glu Glu Lys Ala Tyr Thr Asp Gly Arg Arg Asp

AAT CCT TGT GAA TCT AAC AGA GGA TAT GGG GAT TAC ACA CCA CTA
Asn Pro Cys Glu Ser Asn Arg Gly Tyr Gly Asp Tyr Thr Pro Leu

CCA GCT GGC TAT GTG ACA AAA GAA TTA GAG TAC TTC CCA GAA ACC
Pro Ala Gly Tyr Val Thr Lys Glu Leu Glu Tyr Phe Pro Glu Thr

GAT AAG GTA TGG ATT GAG ATC GGA GAA ACG GAA GGA ACA TTC ATT
Asp Lys Val Trp Ile Glu Ile Gly Glu Thr Glu Gly Thr Phe Ile
                                                        (1170)
GTG GAT AGC GTG GAA TTA CTC CTT ATG GAG GAA TAG
Val Asp Ser Val Glu Leu Leu Leu Met Glu Glu ***
                                                        (1181)
```

## Claims

1. Bacillus thuringiensis kurstaki H.D. 562 cells comprising heterologous DNA comprising a) an origin of replication in said B.t. cells, and b) DNA encoding a B.t. operable gene for the expression of a B.t. endotoxin which is a mutant of an endotoxin protein having insecticidal activity against tobacco budworm larva upon ingestion by the insect, the structural gene DNA for said mutant protein characterized by having a DNA portion coding for an amino acid sequence portion having substantial amino acid homology with the 116 amino acid sequence beginning at position m-1 and extending through position m-116 in Table A hereof, said position numbers applying to such homologous sequence independent of any deletions or additions therein compared to said 116 amino acid sequence, and said DNA portion being further characterized by any one or more of the following amino acids being coded for by said DNA at the indicated amino acid reference positions: a) at position m-30 any natural amino acid except Ala; b) at position m-41 any natural amino acid except Met; c) at position m-99 any natural amino acid except Thr; and d) at position m-112 any natural amino acid except Gly.

2. The cells of claim 1 in which the structural gene DNA would hybridize under stringent conditions to a 348 nucleotide oligomer having the nucleotide sequence beginning at position n-1 and extending through position n-348 in Table A thereof.

3. The cells of claim 1 in which the structural gene DNA would hybridize under stringent conditions to DNA having the coding sequence for the 1181 amino acid endotoxin as set forth in Table A hereof.

4. The cells of Claim 1 in which said DNA portion is characterized by any material amino acid at position

28

m-99 except Thr.

5. The cells of Claim 4 in which the amino acid encoded for at position m-99 is Ser.

6. The cells in accord with any of claims 1-5 in which the Bacillus Thuringiensis kurstaki H.D. 562 cells are of the JAVELIN$^R$ substrain.

7. The cells of claim 6 in which the mutant is p 98cl of Table B hereof.

8. An insecticidal composition comprising a mixture of endotoxin proteins expressed at the sporulation stage by cells in accord with claims 1-7.

9. A process for transforming Bacillus thuringiensis (B.t.) host cells with DNA comprising growing said host cells in a hypertonic aqueous medium, subjecting said cells to high voltage pulsed current in the presence of the desired exogenous DNA while maintaining the hypertonic status to effect transformation of the cells by said DNA, and incubating the thus transformed cells in a hypertonic aqueous incubation medium for a time sufficient to obtain intact transformed cells.

10. The process of claim 9 in which the host cells prior to incorporation into said hypertonic aqueous transformation medium are grown to a cell density $OD_{600}$ of from 0.55 to 0.80 and the current is pulsed at a voltage of 2200 to 3000 volts.

11. The process of claim 10 in which the host cells are grown to a cell density $OD_{600}$ of 0.6 to 0.75.

12. The process of claim 10 in which the host cells are transformed with current pulsed at a voltage of 2300 to 2600 volts.

13. The process of claim 10 in which the hypertonic media contain 0.35M to 0.55M per liter of saccharide as means for inducing their hypertonic status.

14. The process of claim 13 in which the hypertonic growth media contains lysozyme in an amount less than that which would render the cells protoplastic.

15. The process of claim 14 in which the lysozyme concentration is from 20 to 300 micrograms per ml. of the hypertonic aqueous medium.

16. The process of claim 9 in which the DNA is plasmid DNA comprising a DNA gene sequence operable in B.t. and encoding an endotoxin protein, an origin of replication in B.t. and a DNA gene sequence operable in B.t. and encoding anti-biotic resistance.

17. The process of claim 9 in which the host cells are B.t. kurstaki cells.

18. The process of claim 9 in which the host cells are B.t. tenebrionis cells.

19. The process of claim 9 in which the host cells are B.t. aizawai cells.

20. Bacillus thuringiensis tenebrionsis cells transformed with heterologous DNA comprising DNA encoding a B.t. operable gene for a B.t. endotoxin-like protein and an origin of replication operable in B.t.

21. The cells of Claim 20 in which said heterologous DNA additionally comprises an origin of replication operable in E. coli and DNA for expressing anti-biotic resistance in B.t. and E. coli.

22. Bacillus thuringiensis aizawai cells transformed with heterologous DNA comprising DNA encoding a B.t. operable gene for a B.t. endotoxin-like protein and an origin of replication operable in B.t.

23. The cells of Claim 22 in which said heterologous DNA additionally comprises an origin of replication operable in E. coli and DNA for expressing antibiotic resistance in B.t. and E. coli.

24. The cells of Claim 22 in which the encoded endotoxin-like protein comprises the active toxin portion of the endotoxin-like protein encoded for by the plasmid pES-1, or mutant of said encoded pES-1 protein providing a toxicity against both H. virescens and exigua that is greater than the B.t. aizawai cells prior to transformation.

25. The cells of Claim 24 in which the inactive portion of the encoded endotoxin-like protein comprises an amino acid sequence corresponding to an amino acid sequence of the inactive portion of B.t. wuhanensis.

26. The cells of Claim 22 in which the encoded endotoxin-like protein has beginning at its N-terminus thee first 723 amino acids of the endotoxin encoded by pES-1 including all of the active portion of the endotoxin of pES-1.

27. The cells of Claim 22 in which the cells prior to transformation are B.t. aizawai HD-137 cells.

28. The cells of Claim 20 in which the encoded endotoxin-like protein comprises the active toxin portion of the endotoxin-like protein encoded for by the plasmid pES-1.

29. In a process for transforming Bacillus thuringiensis (B.t.) host cells with DNA amplified in bacterial cells which are of a different species, subspecies or strain than such host cells, the improvement in increasing the frequency of such transformation comprising the steps of:
   1) recovering the DNA transformed into such cells which are of a different species, subspecies or strain;
   2) transforming the recovered DNA into B.t. cells of the same subspecies or strain as the host cells;
   3) recovering the DNA transformed in Step 2; and
   4) transforming the host cells with the DNA recovered in Step 3 or a mutant of such DNA.

# FIG. 1

pUCBiB
(5.7 kb)

Bam HI — Sma I

AMP$^R$ — TET$^R$

pUC18
(E.coli) — pBC16.1
(Bacillus)

ori — ori

Bam HI

# FIG. 2

Sma I  Sca I  Mlu I

DET

Chlor

pBEV210TL

ori  Pro.  Spe I

## Key

| | |
|---|---|
| ■ | Transcription Termination Loop |
| ↗ | E. coli/B.t. tandem Promoters |
| Chlor | Chloramphenicol resistance |
| DET | B.t. wuh. delta−endotoxin gene |
| ori | E. coli origin of DNA replication |

# FIG. 3

## pBT1000
### (9.7 kb)

# FIG. 4

Data from "% Survival of cryB"

# FIG. 5

FIG. 6

pBT2000
(9.7 kb)